# EUROPEAN PATENT APPLICATION

(11) **EP 4 317 444 A1**
(43) Date of publication of application: **07.02.2024**
(21) Application number: 22778920.3
(22) Date of filing: 29.03.2022
(51) Int. Cl.: C12N 15/85, C12N 15/113, A61K 31/7105, A61P 35/00

(54) **RNA PLASMID DELIVERY SYSTEM AND APPLICATION THEREOF**

(30) Priority: 29.03.2021 CN 202110335617
(71) Applicant: Nanjing University, Nanjing, Jiangsu 210023 (CN)
(72) Inventor: ZHANG, Chenyu, Nanjing, Jiangsu 210023 (CN); CHEN, Xi, Nanjing, Jiangsu 210023 (CN); FU, Zheng, Nanjing, Jiangsu 210023 (CN); LI, Jing, Nanjing, Jiangsu 210023 (CN); ZHANG, Xiang, Nanjing, Jiangsu 210023 (CN); ZHOU, Xinyan, Nanjing, Jiangsu 210023 (CN); ZHANG, Li, Nanjing, Jiangsu 210023 (CN); YU, Mengchao, Nanjing, Jiangsu 210023 (CN); GUO, Hongyuan, Nanjing, Jiangsu 210023 (CN)
(74) Representative: Cabinet Laurent & Charras
(86) International application number: PCT/CN2022/083598
(87) International publication number: WO 2022/206738

(57) **Abstract**

Provided are an RNA plasmid delivery system and an application thereof. The RNA plasmid delivery system comprises a plasmid carrying an RNA fragment required to be delivered; the plasmid can be enriched in an organ tissue of a host, and spontaneously forms an exosome containing the RNA fragment in the organ tissue of the host, and therefore can enter and be combined with a target tissue to deliver the RNA fragment into the target tissue. The RNA delivery system is safe, reliable, good in druggability, and strong in universality.

## Description

### FIELD

The present application relates to the technical field of biomedicine, and specifically relates to an RNA plasmid delivery system and an application thereof.

### BACKGROUND

RNA interference (RNAi) therapy has been considered a promising strategy to treat human diseases since its invention. However, it has faced numerous issues during clinical practice, and the progress of the therapy's development has fallen short of expectations.

It is generally believed that RNA cannot remain stable outside the cell for a long time, because RNA will be degraded into fragments due to the abundance of RNase in the extracellular environment. It is therefore crucial to find a method that can stabilize RNA outside the cell and enable it to enter targeted tissues, thereby enhancing the effect of RNAi therapy.

There are currently several patents concerning siRNA, mainly focusing on the following aspects: 1. Designing siRNA for medical application. 2. Chemically modifying siRNA in order to enhance its stability *in vivo* and increase the yield. 3. Refining the design of various artificial carriers, including lipid nanoparticles, cationic polymers and plasmids, to improve the efficiency of siRNA delivery *in vivo.* Among them, there are a number of patents regarding the third aspect. The primary cause for this is that researchers have realized the absence of suitable siRNA delivery systems for safely, accurately and efficiently deliver siRNA to target tissues. This challenge has become the fundamental limitation of RNAi therapy.

Chinese patent CN108624590A discloses an siRNA capable of inhibiting the expression of DDR2 gene. Chinese patent CN108624591A discloses an siRNA capable of silencing ARPC4 gene, and the siRNA is modified with α-phosphorus-selenium. Chinese patent CN108546702A discloses an siRNA targeting a long non-coding RNA, DDX11-AS1. Chinese patent CN106177990A discloses an siRNA precursor that can be used for the treatment of various tumors. These patents devise specific siRNAs and target certain diseases caused by genetic mutations.

Chinese patent CN108250267A discloses a polypeptide and a polypeptide-siRNA induced co-assembly, where the polypeptide acts as a carrier of siRNA. Chinese patent CN108117585A discloses a polypeptide to target and to introduce siRNA for promoting apoptosis of breast cancer cells, also utilizing the polypeptide as a carrier of siRNA. Chinese patent CN108096583A discloses a nanoparticle carrier, which can be loaded with siRNA that has curative effect on breast cancer while containing chemotherapeutic drugs. These patents are all inventions on siRNA carriers, whose technical solutions share the common feature of pre-assembling the carrier and siRNA *in vitro* before introducing them to the host. In fact, this is characteristic for most of the currently designed delivery techniques. However, these delivery systems pose a common issue that such artificially synthesized exogenous delivery system is prone to be cleared by the host's circulatory system, cause immunogenic reactions, and even potentially be toxic to certain cell types and tissues.

The research team of the present invention found that endogenous cells can selectively encapsulate miRNAs into exosomes. Exosomes can deliver miRNA to receptor cells, and the secreted miRNA can effectively block the expression of target genes at a relatively low concentration. Exosomes are biocompatible with the host immune system and possess the inherent ability to protect and transport miRNA across biological barriers in vivo, thereby having the potential to overcome problems associated with siRNA delivery. For example, Chinese patent CN110699382A discloses a method for preparing exosomes delivering siRNA, which involves techniques of isolating exosomes from plasma and encapsulating siRNA into exosomes by electroporation.

However, such techniques for isolating or preparing exosomes in vitro often necessitate obtaining a large amount of exosomes through cell culture, together with the step of siRNA encapsulation, which makes the clinical cost of large-scale application of the product too high for patients to afford. More importantly, the intricate production/purification process of exosomes makes it almost impossible to comply with GMP standards.

So far, medicinal products containing exosomes as active ingredients have not received approval from CFDA. The main challenge lies in ensuring the consistency of exosome products, which results in these products failing to obtain drug production licenses. If this issue can be resolved, it would significantly advance RNAi therapy.

Therefore, the development of a safe, precise and efficient siRNA delivery system is a crucial part to improve the efficacy of RNAi therapy and advance it further.

### SUMMARY

In view of this, the embodiments of the present application provide an RNA plasmid delivery system and a application thereof to solve the technical deficiencies existing in the existing technology.

The present application provides an RNA plasmid delivery system, wherein the system comprises a plasmid carrying a RNA fragment required to be delivered, the plasmid can be enriched in an organ tissue of a host, and spontaneously forms a complex structure containing the RNA fragment in the organ tissue of the host, the complex structure can enter and be combined with a target tissue to deliver the RNA fragment into the target tissue. After RNA fragments are introduced into the target tissue, they can inhibit the expression of their matching genes, thereby inhibiting the development of diseases in the target tissue.

Optionally, the RNA fragment comprises one, two or more specific RNA sequences of medical significance, which are siRNA, shRNA, or miRNA sequences of medical significance.

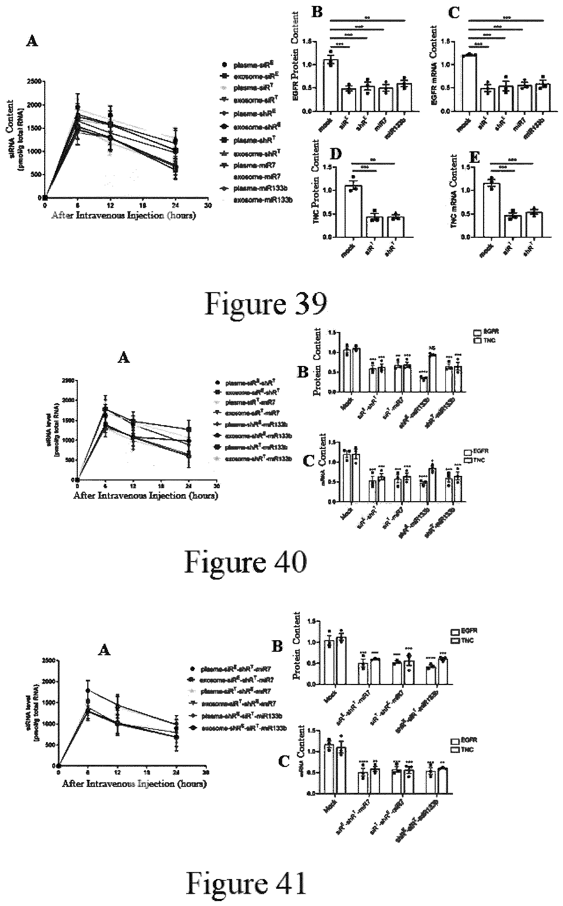

Figures 39-41 show that the constructed plasmids exhibit in vivo enrichment, self-assembly, and disease treatment effects when multiple RNA fragments are used individually, or as a combination for any two or any three.

Optionally, the plasmid further comprises a promoter and a targeting tag, the targeting tag can form a targeting structure of the complex structure in the organ tissue of the host, the complex structure can search for and be combined with the target tissue through the targeting structure to deliver the RNA fragment into the target tissue.

Optionally, the plasmid comprises any one or a combination of the following circuits: promoter-RNA fragment, promoter-targeting tag, promoter-RNA fragment-targeting tag; wherein each of the plasmid comprises at least one RNA fragment and one targeting tag, the RNA fragment and targeting tag are located in the same circuit or in different circuits.

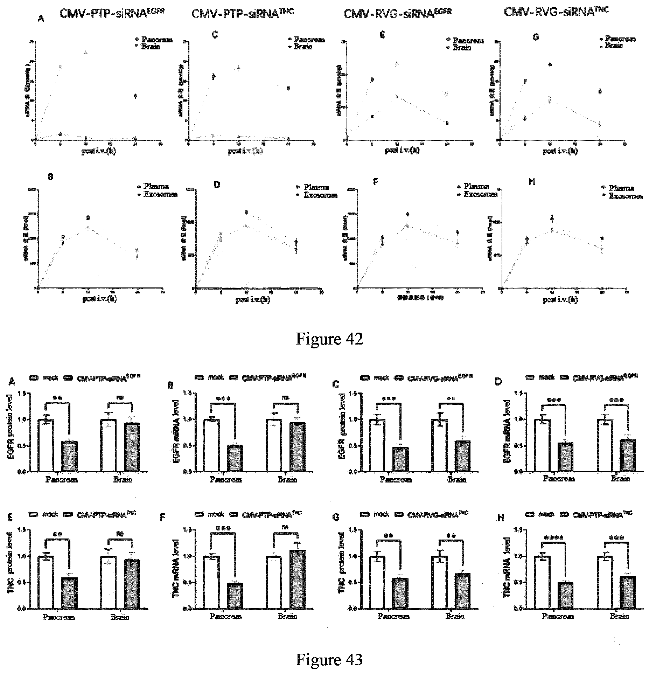

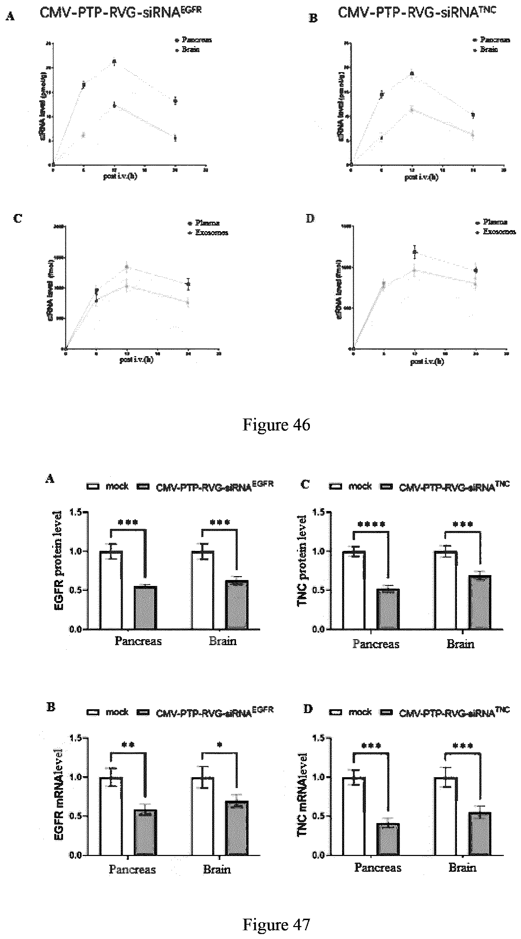

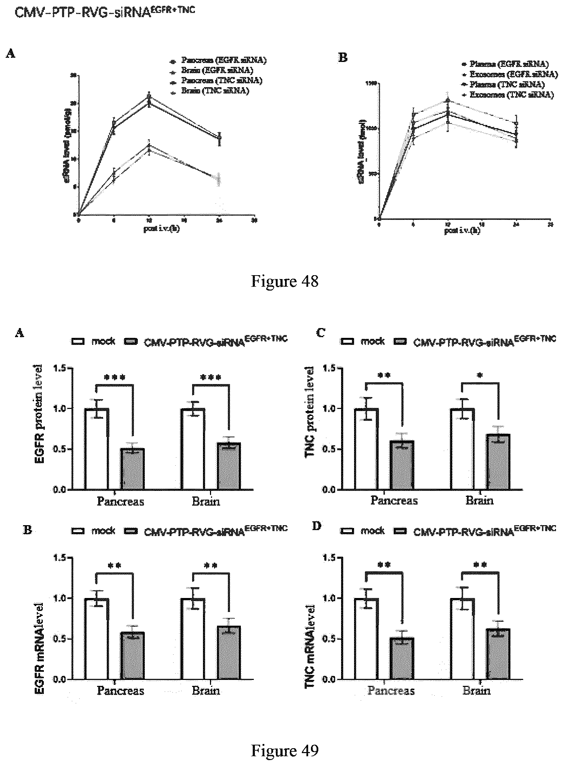

Figures 42-49 show that the delivery system constructed by arbitrarily combination of 2 different RNA fragments with 2 different targeting tags has in vivo enrichment and therapeutic effects.

Optionally, the plasmid further comprises a flanking sequence, a compensation sequence, and a loop sequence that facilitate the correct folding and expression of the circuit, wherein the flanking sequence comprises a 5' flanking sequence and a 3' flanking sequence;
the plasmid comprises any one or a combination of the following circuits: 5' promoter-5' flanking sequence-RNA sequence-loop sequence-compensation sequence-3' flanking sequence, 5'-promoter-targeting tag, and 5' promoter-targeting tag-5' flanking sequence-RNA sequence-loop sequence-compensation sequence-3' flanking sequence.

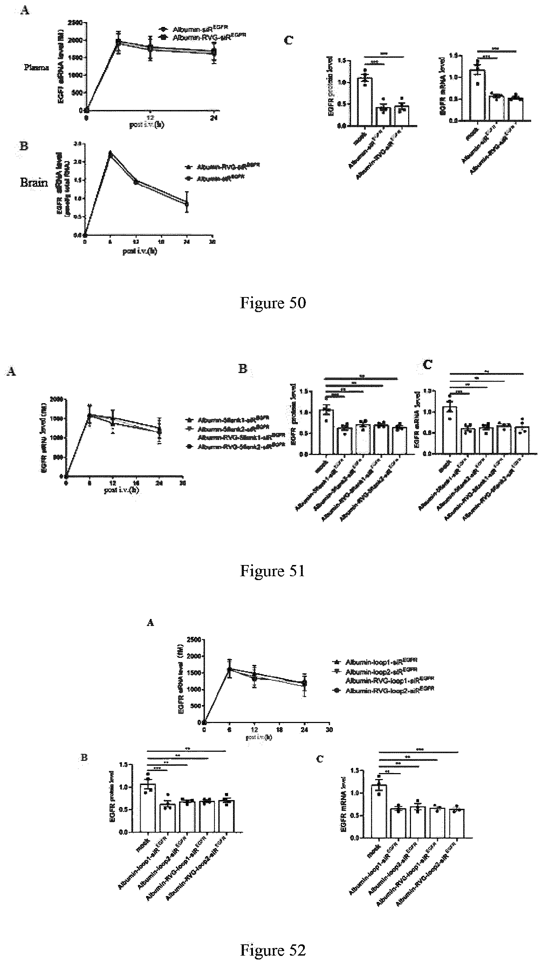

Figure 50 shows the enrichment effect of the circuit of promoter-siRNA and promoter-targeting tag-siRNA gene, as well as the expression level detection results of EGFR.

Optionally, the 5' flanking sequence has a sequence that is identical to or more than 80% homologous with ggatcctggaggcttgctgaaggctgtatgctgaattc;
the loop sequence has a sequence that is identical to or more than 80% homologous with gttttggccactgactgac;
the 3' flanking sequence has a sequence that is identical to or more than 80% homologous with accggtcaggacacaaggcctgttactagcactcacatggaacaaatggcccagatctggccgcactcgag; and
the compensation sequence has a reverse complementary sequence to the RNA fragment in which any 1 to 5 bases have been deleted, so that the compensation sequence is not expressed.

Figures 51-53 show that all of the plasmids constructed by a 5' flanking sequence, a loop sequence, a 3' flanking sequence, or their homologous sequences, have enrichment and therapeutic effects.

Preferably, the compensation sequence has a reverse complementary sequence to the RNA fragment in which any 1 to 3 bases have been deleted.

More preferably, the compensation sequence has a reverse complementary sequence to the RNA fragment in which any 1 to 3 consecutively arranged bases have been deleted.

Most preferably, the compensation sequence has a reverse complementary sequence to the RNA fragment in which bases at positions 9 and/or 10 have been deleted.

Optionally, in the presence of at least two circuits in the plasmid, adjacent circuits are connected through a sequence consisted of sequences 1-3 (sequence 1- sequence 2- sequence 3).
wherein sequence 1 is CAGATC, sequence 2 is a sequence consisted of 5-80 bases, and sequence 3 is TGGATC. Preferably, sequence 2 is a sequence consisted of 10-50 bases, and more preferably, sequence 2 is a sequence consisted of 20-40 bases.

Optionally, in the presence of at least two circuits in the plasmid, adjacent circuits are connected through sequence 4 or a sequence with homology greater than 80% with sequence 4;
wherein, sequence 4 is CAGATCTGGCCGCACTCGAGGTAGTGAGTCGACCAGTGGATC.

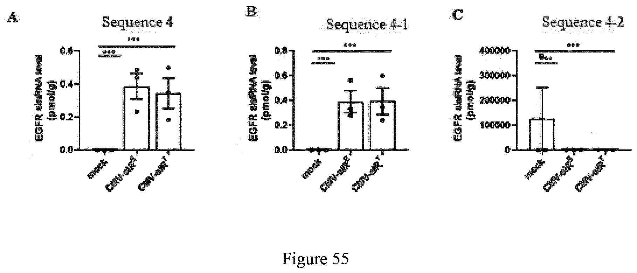

Figure 55 shows that the delivery system constructed by sequence 4 and the homologous sequences thereof have enrichment and therapeutic effects. Optionally, the plasmid is composed of multiple plasmids with different structures, one of which contains a promoter and a targeting tag, while the other plasmids contain a promoter and a RNA sequence.

Optionally, the organ tissue is liver, and the complex structure is an exosome.

Optionally, the targeting tag is selected from a targeting peptide or protein with targeting function, and the targeted structure is located on the surface of the complex structure.

Optionally, the targeted peptide is selected from the group consisting of RVG targeting peptide, GE11 targeting peptide, PTP targeting peptide, TCP-1 targeting peptide, and MSP targeting peptide;
the targeting protein is selected from the group consisting of RVG-LAMP2B fusion protein, GE11-LAMP2B fusion protein, PTP-LAMP2B fusion protein, TCP-1-LAMP2B fusion protein, and MSP-LAMP2B fusion protein.

Optionally, the RNA required to be delivered is 15-25 nucleotides (nt) in length. For example, the RNA sequence can be 16, 17, 18, 19, 20, 21, 22, 23, 24, and 25 nucleotides in length. Preferably, the RNA sequence is 18-22 nucleotides in length.

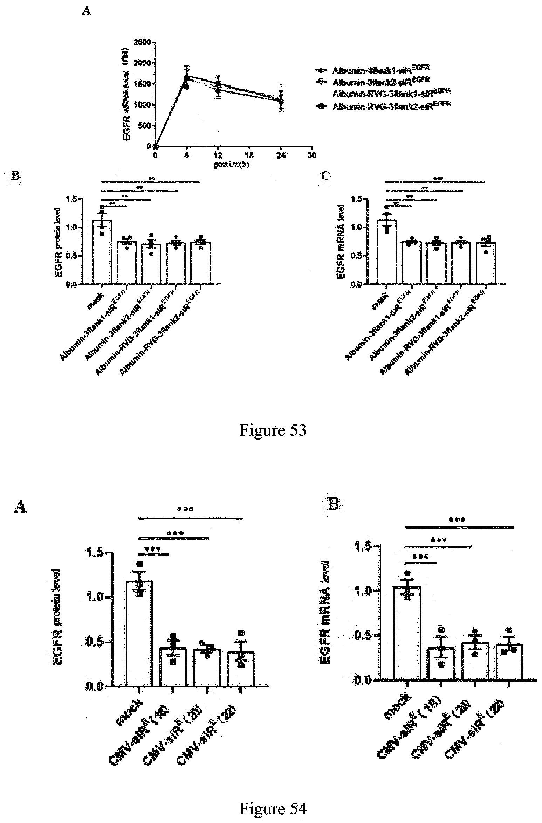

Figure 54 shows that the delivery system (plasmid) constructed by RNA sequences of different lengths exhibit in vivo enrichment and therapeutic effects after intravenous injection.

Optionally, the RNA required to be delivered has a sequence that is identical to, more than 80% homologous with, or of a nucleic acid molecule encoding a sequence selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, siRNA of α-synuclein gene, and a combination thereof. It should be noted that the RNA sequence in "a nucleic acid molecule encoding a sequence selected from" the following herein also includes RNA sequences with more than 80% homology to the above-mentioned RNA sequence.

Wherein, the siRNAs of the above-mentioned genes are RNA sequences that have the function of inhibiting the expression of the gene. Numerous RNA sequences with this function exist and cannot all be listed here, and the following sequences with better effects are provided as examples.

The siRNA of the EGFR gene has a sequence that is identical to or more than 80% homologous with UGUUGCUUCUCUUAAUUCCU, AAAUGAUCLTUCAAAAGUGCCC, UCUUUAAGAAGGAAAGAUCAU, AAUAUUCGUAGCAUUUAUGGA, UAAAAAUCCUCACAUAUACUU, or other sequences that inhibit EGFR gene expression.

The siRNA of the KRAS gene has a sequence that is identical to or more than 80% homologous with UGAULTUAGUALTUAULTUAUGGC, AAUUUGUUCUCUAUAAUGGUG, UAAUUUGUUCUCUAUAAUGGU, UUAUGUUUUCGAAUUUCUCGA, UGUALTCTUACAUAAUUACACAC, or other sequences that inhibit KRAS gene expression.

The siRNA of the VEGFR gene has a sequence that is identical to or more than 80% homologous with ALTCTUGAAGAGLTUGUALTUAGCC, UAAUAGACUGGUAACLTCTUCAU, ACAACUAUGUACAUAAUAGAC, UUUAAGACAAGCUUUUCUCCA, AACAAAAGGUUUUUCAUGGAC, or other sequences that inhibit VEGFR gene expression.

The siRNA of the mTOR gene has a sequence that is identical to or more than 80% homologous with AGAUAGLTUGGCAAAUCUGCCA, ACUALTCTUCAUCCAUAUAAGGU, AAAAUGUUGUCAAAGAAGGGU, AAAAAUGUUGUCAAAGAAGGG, UGALTCTUCLTUCCALTUUCLTUCUC, or other sequences that inhibit mTOR gene expression.

The siRNA of the TNF-α gene has a sequence that is identical to or more than 80% homologous with AAAACAUAAUCAAAAGAAGGC, UAAAAAACAUAAUCAAAAGAA, AAUAAUAAAUAAUCACAAGUG, UUUUCACGGAAAACAUGUCUG, AAACAUAAUCAAAAGAAGGCA, or other sequences that inhibit TNF-α gene expression.

The siRNA of the integrin-α gene has a sequence that is identical to or more than 80% homologous with AUAAUCAUCUCCAUUAAUGUC, AAACAAUUCCUUUUUUAUCUU, AUUAAAACAGGAAACUUUGAG, AUAAUGAAGGAUAUACAACAG, UUCUUUAUUCAUAAAAGUCUC, or other sequences that inhibit integrin-α gene expression.

The siRNA of the B7 gene has a sequence that is identical to or more than 80% homologous with ULTCTUCLTCTUGGGUAAUCLTUCAG, AGAAAAAUUCCACUUUUUCUU, AUUUCAAAGUCAGAUAUACUA, ACAAAAAUUCCAUUUACUGAG, AUUAUUGAGUUAAGUAUUCCU, or other sequences that inhibit B7 gene expression.

The siRNA of the TGF-β1 gene has a sequence that is identical to or more than 80% homologous with ACGGAAAUAACCUAGAUGGGC, UGAACLTUGUCAUAGALTCTUCGU, UUGAAGAACAUAUAUAUGCUG, UCUAACUACAGUAGUGUUCCC, UCUCAGACUCUGGGGCCUCAG, or other sequences that inhibit TGF-β1 gene expression.

The siRNA of the H2-K gene has a sequence that is identical to or more than 80% homologous with AAAAACAAAUCAAUCAAACAA, UCAAAAAAACAAAUCAAUCAA, UAUGAGAAGACAUUGUCUGUC, AACAAUCAAGGUUACAUUCAA, ACAAAACCUCUAAGCALTUCUC, or other sequences that inhibit H2-K gene expression.

The siRNA of the H2-D gene has a sequence that is identical to or more than 80% homologous with AAUCUCGGAGAGACAUUUCAG, AAUGUUGUGUAAAGAGAACUG, AACAUCAGACAAUGUUGUGUA, UGUUAACAAUCAAGGUCACUU, AACAAAAAAACCUCUAAGCAU, or other sequences that inhibit H2-D gene expression.

The siRNA of the H2-L gene has a sequence that is identical to or more than 80% homologous with GAUCCGCUCCCAAUACUCCGG, AUCUGCGUGAUCCGCUCCCAA, UCGGAGAGACAUUUCAGAGCU, UCUCGGAGAGACAUUUCAGAG, AAUCUCGGAGAGACALTCTUCAG, or other sequences that inhibit H2-L gene expression.

The siRNA of the HLA gene has a sequence that is identical to or more than 80% homologous with AUCUGGAUGGUGUGAGAACCG, UGUCACUGCUUGCAGCCUGAG, UCACAAAGGGAAGGGCAGGAA, UUGCAGAAACAAAGUCAGGGU, ACACGAACACAGACACAUGCA, or other sequences that inhibit HLA gene expression.

The siRNA of the GDF15 gene has a sequence that is identical to or more than 80% homologous with UAUAAAUACAGCUGLTUUGGGC, AGACUUAUAUAAAUACAGCUG, AAUUAAUAAUAAAUAACAGAC, AUCUGAGAGCCAUUCACCGUC, UGCAACUCCAGCUGGGGCCGU, or other sequences that inhibit GDF15 gene expression.

The siRNA of the TNC gene has a sequence that is identical to or more than 80% homologous with AUGAAAUGUAAAAAAAGGGA, AAUCAUAUCCUUAAAAUGGAA, UAAUCAUAUCCUUAAAAUGGA, UGAAAAAUCCUUAGUUUUCAU, AGAAGUAAAAAACUALTUGCGA, or other sequences that inhibit TNC gene expression.

The siRNA of the PTP1B gene has a sequence that is identical to or more than 80% homologous with UGAUAUAGUCALTUAUCLTUCLTU, UCCALTCTCTUUAUCAAACUAGCG, AUUGUUUAAAUAAAUAUGGAG, AAUUUUAAUACAUUAUUGGUU, UUUAUUAUUGUACUUUUUGAU, or other sequences that inhibit PTP1B gene expression.

The siRNA of the mHTT gene has a sequence that is identical to or more than 80% homologous with UAUGLTCTCTUCACAUALTUGUCAG, ALTCTUAGUAGCCAACUAUAGAA, AUGUUUUUCAAUAAAUGUGCC, UAUGAAUAGCAUUCUUAUCUG, UAULTUGLTUCCUCLTUAAUACAA, or other sequences that inhibit mHTT gene expression.

The siRNA of the Lrrk2 gene has a sequence that is identical to or more than 80% homologous with AUUAACAUGAAAAUAUCACUU, UUAACAAUAUCAUAUAAUCUU, AUCUUUAAAAUUUGUUAACGC, UUGAUUUAAGAAAAUAGUCUC, UUUGAUAACAGUAUUUUUCUG, or other sequences that inhibit Lrrk2 gene expression.

The siRNA of the α-synuclein gene has a sequence that is identical to or more than 80% homologous with AUAUALTUAACAAALTUUCACAA, AAGUALTUAUAUAUAUUAACAA, AUAACUUUAUAUUUUUGUCCU, UAACUAAAAAAUUAUUUCGAG, UCGAAUALTUALTCTUAUUGUCAG, or other sequences that inhibit α-synuclein gene expression.

It should be noted that the above-mentioned "more than 80% homologous with" a sequence may refer to a homology of 85%, 88%, 90%, 95%, 98%, etc.

Optionally, the RNA fragment includes the RNA sequence and a modified RNA sequence obtained by ribose modification to the RNA sequence. That is to say, the RNA fragment may consist of at least one RNA sequence, at least one modified RNA sequence, or the RNA sequence and the modified RNA sequence.

In the present invention, the isolated nucleic acid also includes variants and derivatives thereof. Those skilled in the art can use common methods to modify the nucleic acid. Such modification includes (but not limited to) methylation modification, hydrocarbon modification, glycosylation modification (such as 2-methoxy-glycosyl modification, hydroxyl-glycosyl modification, saccharide ring modification), nucleic acid modification, peptide fragment modification, lipid modification, halogen modification, and nucleic acid modification (such as "TT" modification). In one embodiment of the present invention, the modification is an internucleotide linkage, for example selected from the group consisting of phosphorothioate, 2'-O methoxyethyl (MOE), 2'-fluoro, alkyl phosphonate, phosphorodithioate, alkyl phosphonothioate, phosphoramidate, carbamate, carbonate, phosphotriester, acetamidate, carboxymethyl ester, and combinations thereof. In one embodiment of the present invention, the modification is a modification of nucleotides, for example, selected from the group consisting of peptide nucleic acid (PNA), locked nucleic acid (LNA), arabinose nucleic acid (FANA), analogs and derivatives thereof, and combinations thereof. Preferably, the modification is 2' fluoropyrimidine modification. 2' Fluoropyrimidine modification is to replace the 2'-OH of the pyrimidine nucleotide of RNA with 2'-F. 2'-F modification can make RNA difficult to be recognized by RNase in vivo, thereby increasing the stability of RNA fragment during transportation in vivo.

Optionally, the delivery system can be used in a mammal including human.

The present application also provides use of the RNA delivery system as described in any paragraph above in the manufacture of a medicament for treating a disease.

Optionally, the medicament is administered by oral administration, inhalation, subcutaneous injection, intramuscular injection, or intravenous injection.. Intravenous injection is preferred.

Optionally, the disease is a cancer, pulmonary fibrosis, colitis, obesity, cardiovascular disease caused by obesity, type 2 diabetes, Huntington's disease, Parkinson's disease, myasthenia gravis, Alzheimer's disease, or graft-versus-host disease.

Optionally, the medicament comprises the aforementioned plasmids, specifically, the plasmids herein represent the plasmids carrying RNA fragments, or carrying RNA fragments and targeting tags, which can enter into the host, accumulate in the liver, self-assemble to form an exosome with a complex structure, in which the complex structure can deliver the RNA fragments to a target tissue, so that the RNA fragments can be expressed in the target tissue, thereby inhibiting the expression of their matching genes for achieving the purpose of disease treatment.

The dosage form of the medicament may be tablets, capsules, powders, granules, pills, suppositories, ointments, solutions, suspensions, lotions, gels, pastes, etc.

**The technical effects of this application include:**

The RNA delivery system provided in the application uses a plasmid as a carrier. As a mature injectable, the safety and reliability of the plasmid have been fully verified, and the druggability of which is proved excellent. The final effective RNA sequence is encapsulated and transported by endogenous exosomes without any immune response, and there is no need to verify the safety of the exosomes. This delivery system can deliver various kinds of small molecule RNA with high versatility. In addition, the preparation of the plasmid is cheaper than the preparation of exosomes or proteins, polypeptides and other substances. Moreover, the RNA delivery system provided by this application can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

The use of the RNA delivery system provided in this application in the medicament offers a platform of drug delivery that facilitates the establishment of further foundations for RNA drug research and development. This significantly advances the development and utilization of RNA drugs.

### BRIEF DESCRIPTION OF DRAWINGS

Figure 1 shows the comparison of plasmid distribution and metabolism in mice provided by an embodiment of the present application.
Figure 2 shows the comparison of protein expression levels in mice provided by an embodiment of the present application.
Figure 3 shows the comparison of related siRNA levels in mice provided by an embodiment of the present application.
Figure 4 shows the comparison of absolute siRNA levels in various tissues of mice provided by an embodiment of the present application.
Figure 5 shows the comparison of the impact of plasmid doses on mouse siRNA levels provided by an embodiment of the present application.
Figure 6 shows the comparison of the metabolism of precursors and mature bodies in the mouse liver after plasmid injection provided by an embodiment of the present application.
Figure 7 shows the comparison of siRNA kinetics and distribution in different tissues of mice provided by an embodiment of the present application.
Figure 8 shows the comparison of the influence of different promoters on siRNA provided by an embodiment of the present application.
Figure 9 shows the comparison of eGFP fluorescence intensity in different tissues of mice provided by an embodiment of the present application.
Figure 10 shows the comparison of mouse alanine aminotransferase, aspartate aminotransferase, total bilirubin, blood urea nitrogen, serum alkaline phosphatase, creatinine contents, thymus weight, spleen weight, and percentage in peripheral blood cells provided by an embodiment of the present application.
Figure 11 shows the comparison of the therapeutic effects of mouse EGFR mutant lung cancer tumors provided by an embodiment of the present application.
Figure 12 shows mouse HE staining, immunohistochemical staining and staining statistics provided by an embodiment of the present application.
Figure 13 shows the comparison of the therapeutic effects of mouse KRAS mutant lung cancer tumors provided by an embodiment of the present application.
Figure 14 shows mouse HE staining, immunohistochemical staining and staining statistics provided by an embodiment of the present application.
Figure 15 shows the comparison of kidney cancer tumors in mice provided by an embodiment of the present application.
Figure 16 shows the comparison of the development of renal cancer tumors in mice provided by an embodiment of the present application.
Figure 17 shows the comparison of the development of colitis in mice provided by an embodiment of the present application.
Figure 18 shows the comparison of colon HE staining in mice provided by an embodiment of the present application.
Figure 19 shows the comparison of the development of colitis in mice provided by an embodiment of the present application.
Figure 20 shows the comparison of colon HE staining in mice provided by an embodiment of the present application.
Figure 21 shows the comparison of hydroxyproline content in mice provided by an embodiment of the present application.
Figure 22 is a fluorescence staining image of the lungs in mice provided by an embodiment of the present application.
Figure 23 is a Masson trichrome staining diagram of the lungs in mice provided by an embodiment of the present application.
Figure 24 is a HE staining image of the lungs in mice provided by an embodiment of the present application.
Figure 25 shows the comparison of some protein and mRNA levels in mice provided by an embodiment of the present application.
Figure 26 shows the comparison of siRNA-related expression in mice provided by an embodiment of the present application.
Figure 27 shows the comparison of the treatment of glioblastoma in mice provided by an embodiment of the present application.
Figure 28 shows the comparison of immunohistochemical staining of mouse brain provided by an embodiment of the present application.
Figure 29 shows fluorescence microscope images of mouse hypothalamus and liver provided by an embodiment of the present application.
Figure 30 shows the comparison of the treatment of obesity in mice provided by an embodiment of the present application.
Figure 31 shows the comparison of the treatment of obesity and fatty liver in mice provided by an embodiment of the present application.
Figure 32 shows the comparison of the treatment of Huntington's disease in mice provided by an embodiment of the present application.
Figure 33 shows the comparison of siRNA and protein levels in mouse liver, cortex, and striatum provided by an embodiment of the present application.
Figure 34 shows the comparison of the treatment of Huntington's disease in mice provided by an embodiment of the present application.
Figure 35 shows the comparison of mHTT protein levels and toxic aggregates in the mouse striatum and cortex provided by an embodiment of the present application.
Figure 36 shows the comparison of the treatment of Parkinson's disease in transgenic mice provided by an embodiment of the present application.
Figure 37 shows siRNA concentration changes in whole blood of Macaca fascicularis provided by an embodiment of the present application.
Figure 38 is an image of the construction and characterization of a circuit provided by an embodiment of the present application.
Figure 39 shows the enrichment effect of plasmids containing 6 different RNAs in plasma, the detection of siRNA in the exosomes and corresponding gene expression levels provided by an embodiment of the present application. In the figure, A represents the enrichment effect of the plasmids in plasma and the detection of siRNA in the exosomes, B and C represent the protein and mRNA expression levels of EGFR, and D and E represent the protein and mRNA expression levels of TNC.
Figure 40 shows the enrichment effect of plasmids containing RNA fragments consisted of any two RNA sequences from six different RNAs in plasma, the detection of siRNA in the exosomes, and corresponding gene expression levels provided by an embodiment of the present application. In the figure, A represents the enrichment effect of the plasmids in plasma and the detection of siRNA in the exosomes, B represents the protein expression levels of EGFR and TNC, and C represents the mRNA expression levels of EGFR and TNC.
Figure 41 shows the enrichment effect of plasmids containing RNA fragments consisted of any three RNA sequences from six different RNAs in plasma, the detection of siRNA in the exosomes, and corresponding gene expression levels provided by an embodiment of the present application. In the figure, A represents the enrichment effect of the plasmids in plasma and the detection of siRNA in the exosomes, B represents the protein expression levels of EGFR and TNC, and C represents the mRNA expression levels of EGFR and TNC.
Figure 42 shows the enrichment effect of the gene circuit comprising two different RNA fragments and two different targeting tags in pancreas, brain, plasma and exosomes provided by an embodiment of the present application; where A and B shows the enrichment effect of the gene circuit with siR^{EGFR} as the RNA fragment and PTP as the targeting tag, C and D shows the enrichment effect of the gene circuit with siR^{TNC} as the RNA fragment and PTP as the targeting tag, E and F shows the enrichment effect of the gene circuit with siR^{EGFR} as the RNA fragment and RVG as the targeting tag, and G and H shows the enrichment effect of the gene circuit with siR^{TNC} as the RNA fragment and RVG as the targeting tag.
Figure 43 shows the expression levels of EGFR and TNC detected in the pancreas and brain with the gene circuit comprising two different RNA fragments and two different targeting tags provided by an embodiment of the present application; where A and B show the protein and mRNA expression levels of EGFR with the gene circuit with siR^{EGFR} as the RNA fragment and PTP as the targeting tag, C and D show the protein and mRNA expression levels of EGFR with the gene circuit with siR^{EGFR} as the RNA fragment and RVG as the targeting tag, E and F show the protein and mRNA expression levels of EGFR with siR^{TNC} as the RNA fragment and PTP as the targeting tag, and G and H show the protein and mRNA expression levels of EGFR with the gene circuit with siR^{TNC} as the RNA fragment and RVG as the targeting tag.
Figure 44 shows the enrichment effect of the gene circuit comprising two RNA fragments and two different targeting tags in the pancreas, brain, plasma, and exosomes provided by an embodiment of the present application; where A and B shows the enrichment effect of the gene circuit with siR^{EGFR+TNC} as the RNA fragment and PTP as the targeting tag, and C and D shows the enrichment effect of the gene circuit with siR^{EGFR+TNC} as the RNA fragment and RVG as the targeting tag.

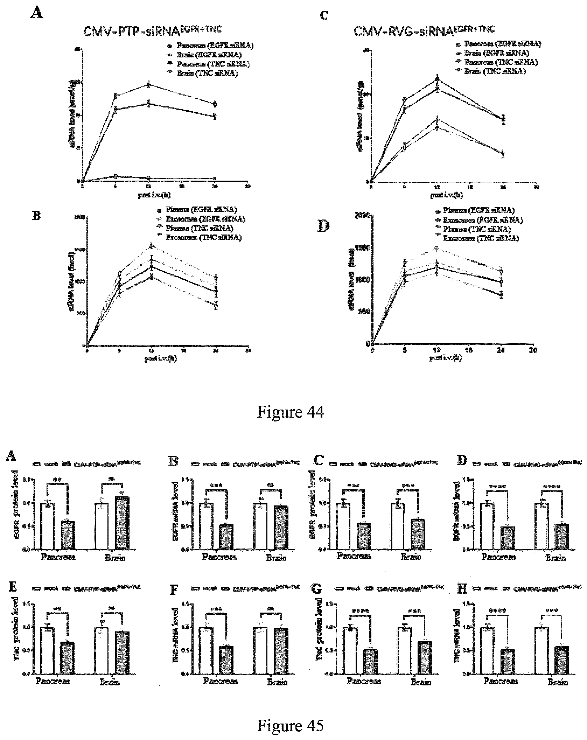

Figure 45 shows the expression levels of EGFR and TNC detected in the pancreas and brain with the gene circuit comprising two RNA fragments and two different targeting tags provided by an embodiment of the present application; where A and B show the protein and mRNA expression levels of EGFR with the gene circuit with siR^{EGFR+TNC} as the RNA fragment and PTP as the targeting tag, C and D show the protein and mRNA expression levels of EGFR with the gene circuit with siR^{EGFR+TNC} as the RNA fragment and RVG as the targeting tag, E and F show the protein and mRNA expression levels of TNC with the gene circuit with siR^{EGFR+TNC} as the RNA fragment and PTP as the targeting tag, and G and H show the protein and mRNA expression levels of TNC with the gene circuit with siR^{EGFR+TNC} as the RNA fragment and RVG as the targeting tag.
Figure 46 shows the enrichment effect of the gene circuit comprising two different RNA fragments and two targeting tags in the pancreas, brain, plasma, and exosomes provided by an embodiment of the present application; where A and B shows the enrichment effect of the gene circuit with siR^{EGFR} as the RNA fragment and PTP-RVG as the targeting tag, and C and D shows the enrichment effect of the gene circuit with siR^{TNC} as the RNA fragment and PTP-RVG as the targeting tag.
Figure 47 shows the expression levels of EGFR and TNC detected in the pancreas and brain with the gene circuit comprising two different RNA fragments and two targeting tags provided by an embodiment of the present application; where A and B show the protein and mRNA expression levels of EGFR with the gene circuit with siR^{EGFR} as the RNA fragment and PTP-RVG as the targeting tag, and C and D show the protein and mRNA expression levels of TNC with the gene circuit with siR^{TNC} as the RNA fragment and PTP-RVG as the targeting tag.
Figure 48 shows the enrichment effect of the gene circuit comprising two RNA fragments and two targeting tags in the pancreas, brain, plasma, and exosomes provided by an embodiment of the present application; where A shows the enrichment effect of the gene circuit with siR^{EGFR+TNC} as the RNA fragment and PTP-RVG as the targeting tag in the pancreas and brain, and B shows the enrichment effect of the gene circuit with siR^{EGFR+TNC} as the RNA fragment and PTP-RVG as the targeting tag in the plasma, and exosomes.
Figure 49 shows the expression levels of EGFR and TNC detected in the pancreas and brain with the gene circuit comprising two RNA fragments and two targeting tags provided by an embodiment of the present application; where A and B show the protein and mRNA expression levels of EGFR with the gene circuit with siR^{EGFR+TNC} as the RNA fragment and PTP-RVG as the targeting tag, and C and D show the protein and mRNA expression levels of TNC with the gene circuit with siR^{EGFR+TNC} as the RNA fragment and PTP-RVG as the targeting tag.
Figure 50 shows the enrichment effect of the two gene circuits of Albumin-siR^{EGFR} and Albumin-RVG-siR^{EGFR} in the plasma and brain and the expression level of EGFR provided by an embodiment of the present application; where A shows the enrichment effect of the two gene circuits in the plasma, B shows the enrichment effect of the two gene circuits in the brain, and C shows the protein and mRNA expression levels of EGFR detected with the two gene circuits.
Figure 51 shows the enrichment effect and therapeutic effect of the delivery system with a more than 80% homologous 5' flanking sequence in the lung provided by an embodiment of the present application; where A shows the enrichment effect of two more than 80% homologous 5' flanking sequences with or without linking RVG in the lung, B shows the protein level of EGFR with two more than 80% homologous 5' flanking sequences with or without linking RVG, and C shows the mRNA level of EGFR with two more than 80% homologous 5' flanking sequences with or without linking RVG.
Figure 52 shows the enrichment effect and therapeutic effect of the delivery system with a more than 80% homologous loop sequence in the lung provided by an embodiment of the present application; where A shows the enrichment effect of two more than 80% homologous loop sequences with or without linking RVG in the lung, B shows the protein level of EGFR with two more than 80% homologous loop sequences with or without linking RVG, and C shows the mRNA level of EGFR with two more than 80% homologous loop sequences with or without linking RVG.
Figure 53 shows the enrichment effect and therapeutic effect of the delivery system with a more than 80% homologous 3' flanking sequence in the lung provided by an embodiment of the present application; where A shows the enrichment effect of two more than 80% homologous 3' flanking sequences with or without linking RVG in the lung, B shows the protein level of EGFR with two more than 80% homologous 3' flanking sequences with or without linking RVG, and C shows the mRNA level of EGFR with two more than 80% homologous 3' flanking sequences with or without linking RVG.
Figure 54 shows the detection results of EGFR expression levels after intravenous injection of three delivery systems comprising the RNA sequence of different lengths provided by an embodiment of the present application; where A shows the detection result of EGFR protein expression levels, and B shows the detection result of EGFR mRNA expression levels.
Figure 55 shows the EGFR siRNA content (enrichment) detection results in lung tissue after intravenous injection of the delivery system comprising sequence 4 or two sequences 4-1 and 4-2 with more than 80% homology to sequence 4 provided by an embodiment of the present application; where A shows the detection result of sequence 4, B shows the detection result of sequence 4-1, and C shows the detection result of sequence 4-2.

### DETAILED DESCRIPTION

Specific embodiments of the present application will be described below in conjunction with the accompanying drawings.

First, the technical terms, experimental methods, etc. involved in the present invention are explained.

Hematoxylin-eosin staining, referred to as HE staining, is one of the most basic and widely used technical methods in the teaching and scientific research of histology and pathology.

Hematoxylin stain is alkaline and can stain the basophilic structures of tissues (such as ribosomes, nuclei and ribonucleic acid in the cytoplasm, etc.) into blue-purple, and eosin is an acidic dye that can stain eosinophilic structures of tissues (such as intracellular and intercellular proteins, Lewy bodies, Mallory bodies, and most of the cytoplasm) into pink, making the morphology of the entire cell tissue clearly visible.

The specific steps of HE staining include: sample tissue fixation and sectioning; tissue sample deparaffinizing; tissue sample hydration; hematoxylin staining of tissue sections, differentiation and anti-blue; eosin staining of tissue sections and dehydration; air-drying and sealing tissue sample sections; and finally, observation and taking pictures under a microscope.

Masson's staining makes collagen fibers blue (stained by aniline blue) or green (stained by bright green), and muscle fibers red (stained by acid fuchsin and ponceau), and is related to the size of the anionic dye molecules and the permeability of the tissue. The fixated tissue is stained sequentially or mixed with a series of anionic water-soluble dyes. It can be found that red blood cells are stained by the smallest molecular anionic dyes, muscle fibers and cytoplasm are stained by medium-sized anionic dyes, and collagen fibers are stained by macromolecular anionic dyes. This shows that red blood cells are the least permeable to anionic dyes, followed by muscle fibers and cytoplasm, while collagen fibers have the greatest permeability. Type I and type III collagen are green (GBM, TBM, mesangial matrix and renal interstitium are green), and eosinophilic proteins, renal tubular cytoplasm and red blood cells are red.

The specific steps of Masson's staining include:
Fixing the tissue in Bouin's solution, rinsing with running water overnight, and routinely dehydrating and embedding; deparaffinizing and dehydrating sections (deparaffinizing in xylene for 10 min× 3 times, absorbing the liquid with absorbent paper; 100% ethanol for 5 min × 2 times, absorbing the liquid with absorbent paper; 95% ethanol for 5 min × 2 times, absorbing the liquid with absorbent paper; rinsing with running water for 2 min, absorbing the liquid with absorbent paper); staining with Weigert's iron hematoxylin for 5-10 min; washing slightly with running water; differentiating with 0.5% hydrochloric acid and alcohol for 15 seconds; rinsing with running water for 3 min; staining with ponceau-fuchsin solution for 8 min; rinsing slightly with distilled water; treating with 1% phosphomolybdic acid aqueous solution for about 5 min; directly counterstaining with aniline blue solution or bright green solution for 5 min without washing with water; treating with 1% glacial acetic acid for 1 min; dehydrating in 95% ethanol for 5 min × 2 times, absorbing the liquid with absorbent paper; 100% ethanol for 5 min × 2 times, absorbing the liquid with absorbent paper; clearing in xylene for 5 min × 2 times, absorbing the liquid with absorbent paper; and sealing with neutral gum.

Western Blot transfers proteins to a membrane and then uses antibodies for detection. For known expressed proteins, the corresponding antibodies can be used as primary antibodies for detection. For the expression products of new genes, the antibodies against the fusion part can be used for detection.

In Western Blot, polyacrylamide gel electrophoresis is employed, where the detected object is protein, the "probe" is an antibody, and the "color development" uses a labeled secondary antibody. The protein sample separated by PAGE is transferred to a solid-phase carrier (such as nitrocellulose film), where it is absorbed through non-covalent bonds without changing the type or biological activity of the polypeptide separated by electrophoresis. The protein or peptide on the solid-phase carrier acts as an antigen and reacts immunologically with the corresponding antibody. Then, it reacts with the second antibody labeled with either enzyme or isotope. The protein component expressed by the specific target gene is separated through electrophoresis and detected through substrate color development or autoradiography. The process primarily comprises of extracting proteins, quantifying proteins, preparing gels, running electrophoresis, transferring membranes, performing immunolabeling, and developing.

Immunohistochemistry (also known as immunocytochemistry), based on the antigen-antibody reaction, i.e., the principle of specific binding of antigen and antibody, is the process of identifying antigens (polypeptides or proteins) in tissue cells, determining their localization, and studying them qualitatively and relative quantitatively by chemical reaction of the chromogenic agent (fluorescein, enzyme, metal ion, or isotope) of the labeled antibody to develop color.

The main steps of immunohistochemistry include soaking sections, airing overnight, deparaffinizing in xylene followed by alcohol of gradient concentrations (100%, 95%, 90%, 80%, 75%, 70%, 50%, 3 min each time), using double distilled water, adding dropwise 3% hydrogen peroxide solution to remove catalase, washing with water, repairing antigen, adding dropwise 5% BSA, blocking for 1h, diluting primary antibody, washing with PBS buffer, incubating with secondary antibody, washing with PBS buffer, developing color with chromogenic solution, washing with water, staining with hematoxylin, dehydrating with ethanol of gradient concentrations, and sealing with neutral gum.

The detection of siRNA, protein and mRNA levels involved in the present invention is all performed by injecting an RNA delivery system into mice to establish an in vitro mouse stem cell model. qRT-PCR is used to detect the expression levels of mRNA and siRNA in cells and tissues. The absolute quantification of siRNA is determined by drawing a standard curve with a standard product. The expression level of each siRNA or mRNA relative to the internal reference can be represented by 2-ΔCT, where ΔCT=Csample-Cinternal reference. The internal reference gene used is U6 snRNA (in tissue) or miR-16 (in serum, exosomes) molecules when amplifying siRNA, and is GAPDH or 18s RNA when amplifying mRNA. Western blotting is used to detect the expression level of proteins in cells and tissues, and ImageJ software is used for protein quantitative analysis.

In the present invention, unless otherwise specified, the scientific and technical terms used herein have the meanings commonly understood by those skilled in the art. Moreover, the reagents, materials and procedures used herein are all reagents, materials and procedures widely used in the corresponding fields.

### Example 1

In this example, an RNA plasmid delivery system is provided, which comprises a plasmid carrying a RNA fragment required to be delivered, the plasmid can be enriched in an organ tissue of a host, and spontaneously forms a complex structure containing the RNA fragment in the organ tissue of the host, the complex structure can enter and be combined with a target tissue to deliver the RNA fragment into the target tissue.

In this example, the plasmid further comprises a promoter and a targeting tag. The plasmid comprises any one or a combination of the following circuits: promoter-RNA fragment, promoter-targeting tag, promoter-RNA fragment-targeting tag; wherein each of the plasmid comprises at least one RNA fragment and one targeting tag, the RNA fragment and targeting tag are located in the same circuit or in different circuits. In other words, the plasmid can only comprise promoter-RNA sequence-targeting tag, or it can comprise a combination of promoter-RNA sequence and promoter-targeting tag, or a combination of promoter-targeting tag and promoter-RNA sequence-targeting tag.

Figures 42-49 show the detection results of combinations of 2 different RNA fragments and 2 different targeting tags. Specifically, RNA fragment 1 is siR^{EGFR}, RNA fragment 2 is siR^{TNC}, targeting tag 1 is PTP, and targeting tag 2 is RVG. After combining the RNA fragments and targeting tags in every way, the enrichment effect of EGFR siRNA and TNC siRNA in pancreas, brain, plasma and exosomes as well as the expression levels of EGFR and TNC were detected.

Further, the plasmid also can comprise a flanking sequence, a compensation sequence, and a loop sequence that facilitate the correct folding and expression of the circuit, wherein the flanking sequence comprises a 5' flanking sequence and a 3' flanking sequence; and the plasmid comprises any one or a combination of the following circuits: 5' promoter-5' flanking sequence-RNA sequence-loop sequence-compensation sequence-3' flanking sequence, 5'-promoter-targeting tag, and 5' promoter-targeting tag-5' flanking sequence-RNA sequence -loop sequence-compensation sequence-3' flanking sequence.

In Figure 50, Albumin is the promoter, RVG is the targeting tag, and siR^{EGFR} is the RNA fragment targeting the EGFR protein. Since the gene circuit cannot function without RNA fragments, only the enrichment effects of the two gene circuits Albumin-siR^{EGFR} and Albumin - RVG- siR^{EGFR} in plasma and brain and the expression level of EGFR were detected.

Wherein, the 5' flanking sequence preferably has a sequence that is identical to or more than 80% homologous with ggatcctggaggcttgctgaaggctgtatgctgaattc, including sequences with 85%, 90%, 92%, 95%, 98%, or 99% homology with ggatcctggaggcttgctgaaggctgtatgctgaattc.

The loop sequence preferably has a sequence that is identical to or more than 80% homologous with gttttggccactgactgac, including sequences with 85%, 90%, 92%, 95%, 98%, 99% homology with gttttggccactgactgac.

The 3' flanking sequence preferably has a sequence that is identical to or more than 80% homologous with accggtcaggacacaaggcctgttactagcactcacatggaacaaatggcccagatctggccgcactcgag, including sequences with 85%, 90%, 92%, 95%, 98%, 99% homology with accggtcaggacacaaggcctgttactagcactcacatggaacaaatggcccagatctggccgcactcgag.

The compensation sequence is the reverse complementary sequence of the RNA fragment with any 1-5 bases deleted. In the case where the RNA fragment contains only one RNA sequence, the compensation sequence may be the reverse complementary sequence of the RNA sequence with any 1-5 bases deleted.

Preferably, the compensation sequence is the reverse complementary sequence of the RNA fragment with any 1-3 bases deleted. In the case where the RNA fragment contains only one RNA sequence, the compensation sequence may be the reverse complementary sequence of the RNA sequence with any 1-3 bases deleted.

More preferably, the compensation sequence is the reverse complementary sequence of the RNA fragment with any 1-3 contiguous bases deleted. In the case where the RNA fragment contains only one RNA sequence, the compensation sequence may be the reverse complementary sequence of the RNA sequence with any 1-3 contiguous bases deleted.

Most preferably, the compensation sequence is the reverse complementary sequence of the RNA fragment with the base at position 9 and/or 10 deleted. In the case where the RNA fragment contains only one RNA sequence, the compensation sequence may be a reverse complementary sequence of the RNA sequence with the base at position 9 and/or 10 deleted. Deleting the base at position 9 and/or 10 has the best effect.

It should be noted that the above-mentioned flanking sequence, compensation sequence, and loop sequence are not randomly selected, but are determined based on a large number of theoretical studies and experiments. With the cooperation of the above-mentioned certain flanking sequence, compensation sequence, and loop sequence, the expression rate of RNA fragments can be maximized.

Figures 51-53 respectively show the enrichment effect and therapeutic effect of the delivery system (plasmid) constructed with two more than 80% homologous 5' flanking sequence, loop sequence and 3' flanking sequence in the lung. Specifically, Figure 51 shows the enrichment effect and therapeutic effect of the plasmid with a more than 80% homologous 5' flank sequence in the lung, Figure 52 shows the enrichment effect and therapeutic effect of the plasmid with a more than 80% homologous loop sequence in the lung, and Figure 53 shows the enrichment effect and therapeutic effect of the plasmid with a more than 80% homologous 3' flank sequence in the lung.

The above sequences are specifically shown in Table 1 below.

| **Name** | **Sequence** |
|---|---|
| 5 flank1 | ggataatggaggcttgctgcaggctgtatgctgaattc |
| 5flank2 | ggatactggacgcttgcttaaggctgtatggtgaattc |
| Loop1 | gacttggccactgactgac |
| Loop2 | gttttggccactggctgtc |
| 3flank1 | agccgtcaggacatgaggcctgttactagcactcacgtggctcaaatggcagagatctggctacactccag |
| 3flank2 | actggtcacgacacaaggcctattactagcagtcacattgaacaaatggccaagatctcgccgcactgtag |

In the case of the plasmid carrying two or more circuits, adjacent circuits can be connected through sequence 1-sequence 2-sequence 3; wherein sequence 1 is preferably CAGATC, sequence 2 can be a sequence consisted of 5-80 bases, such as 10, 15, 20, 25, 30, 35, 40, 45, 50, 55, 60, 65, 70, or 75 bases, preferably a sequence consisted of 10-50 bases, more preferably a sequence consisted of 20-40 bases, and sequence 3 is preferably TGGATC.

The sequence 2 is shown specifically in Table 2.

| | |
|---|---|
| 20 Base s | TGGCCGCACTCGAGGTAGTG |
| 30 Base s | TGGCCGCACTCGAGGTAGTGAGTCGACCAG |
| 40 Base s | TGGCCGCACTCGAGGTAGTGAGTCGACCAGTGGCCGCACT |
| 50 Base s | TGGCCGCACTCGAGGTAGTGAGTCGACCAGTGGCCGCACTCGAGGTAGTG |
| 60 Base s | TGGCCGCACTCGAGGTAGTGAGTCGACCAGTGGCCGCACTCGAGGTAGTG AGTCGACCAG |
| 70 Base | TGGCCGCACTCGAGGTAGTGAGTCGACCAGTGGCCGCACTCGAGGTAGTG |
| s | AGTCGACCAGTGGCCGCACT |
| 80 Base s | TGGCCGCACTCGAGGTAGTGAGTCGACCAGTGGCCGCACTCGAGGTAGTG AGTCGACCAGTGGCCGCACTCGAGGTAGTG |

More preferably, in the case of the plasmid carrying two or more circuits, adjacent circuits are connected through sequence 4 or a sequence with homology greater than 80% with sequence 4; wherein sequence 4 is CAGATCTTGGCCGCACTCAGGTAGTGAGTCGACCAGTGGATC.

Figure 55 shows the results of detection of the EGFR siRNA content in the lung tissue 9 hours after intravenous injection with the delivery system constructed with sequence 4 and two sequences 4-1 and 4-2 with homology greater than 80% with sequence 4.

The sequence 4 is shown specifically in Table 3.

| **Name** | **Sequence** |
|---|---|
| Sequence 4 | CAGATCTGGCCGCACTCGAGGTAGTGAGTCGACCAGTGGATC |
| Sequence 4-1 | CAGATGATTCCGCACTCGAGGTCATGAGTCGACCAGTGGATC |
| Sequence 4-2 | CAGATCTAAGGTCACTCGAGGTAGTGCTACGACCAGTGGATC |

The aforementioned RNA fragments comprise one, two or more specific RNA sequences with medical significance, which can be expressed in the target receptor, while the compensation sequence cannot be expressed in the target receptor. The RNA sequence may be an siRNA sequence, shRNA sequence or miRNA sequence, preferably an siRNA sequence.

The length of an RNA sequence is 15-25 nucleotides (nt), preferably 18-22 nt, such as 18 nt, 19 nt, 20 nt, 21 nt, or 22 nt. The range of the sequence length is not chosen arbitrarily but was determined after repeated trials. A large number of experiments have proved that in the case where the length of the RNA sequence is less than 18 nt, especially less than 15 nt, the RNA sequence is mostly invalid and will not play a role. In the case where the length of the RNA sequence is greater than 22 nt, especially greater than 25 nt, not only does the cost of the circuit increase greatly, but the effect is no better than that of an RNA sequence with a length of 18-22 nt, and the economic benefits are poor. Therefore, in the case where the length of the RNA sequence is 15-25 nt, especially 18-22 nt, the cost and function can be balanced with the best effect.

RNA sequences of different lengths are shown specifically in Table 4 below.

**Table 4**

| **Name** | **Sequence** |
|---|---|
| siRE(18) | ACCTATTCCGTTACACACT |
| siRE(20) | ATACCTATTCCGTTACACAC |
| siRE(22) | ATACCTATTCCGTTACACACTT |

Figure 54 shows the detection of EGFR expression levels after intravenous injection of delivery systems (plasmids) constructed with RNA sequences of different lengths, where the plasmids with RNA sequences of lengths of 18, 20, and 22 correspond to CMV-siR^{E} (18), CMV-siR^{E} (20), and CMV-siR^{E} (22).

The above-mentioned RNA sequence has a sequence that is identical to, more than 80% homologous with, or of a nucleic acid molecule encoding a sequence selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, siRNA of α-synuclein gene, and a combination thereof.

The siRNAs and the antisense strands of miRNAs of the above-mentioned genes can inhibit the expression or mutation of the genes and miRNAs, thereby achieving the effect of inhibiting diseases. These diseases include, but are not limited to: cancer, pulmonary fibrosis, colitis, obesity, cardiovascular disease caused by obesity, type 2 diabetes, Huntington's disease, Parkinson's disease, myasthenia gravis, Alzheimer's disease, graft-versus-host disease and related diseases. The related diseases herein refer to the associated diseases, complications or sequelae that appear during the formation or development of any one or several of the above diseases, or other diseases that are related to the above diseases to a certain extent. Among them, cancers include but are not limited to: gastric cancer, kidney cancer, lung cancer, liver cancer, brain cancer, blood cancer, bowel cancer, skin cancer, lymphatic cancer, breast cancer, bladder cancer, esophageal cancer, head and neck squamous cell carcinoma, hemangioma, glioblastoma, or melanoma.

RNA fragments can have 1, 2, or more RNA sequences. For example, the siRNA of EGFR gene and that of TNC gene can be combined on the same plasmid vector for treatment of glioma; and the siRNAs of TNF-α gene, integrin-α gene and B7 gene can be used simultaneously for treatment of enteritis.

Taking the combination of "siRNA1" and "siRNA2" on the same plasmid vector as an example, the functional structural region of the plasmid vector can be represented as follows: (promoter-siRNA1)-linker-(promoter-siRNA2)-linker-(promoter-targeting tag), or (promoter-targeting tag-siRNA1)-linker-(promoter-targeting tag-siRNA2), or (promoter-siRNA1)-linker-(promoter-targeting tag-siRNA2), etc..

More specifically, the functional structural region of the delivery carrier can be expressed as (5'-promoter-5' flanking sequence-siRNA 1-loop sequence-compensation sequence-3' flanking sequence)-linker-(5'-promoter-5'flanking sequence-siRNA 2-loop sequence-compensation sequence-3' flanking sequence)-linker-(5'-promoter-targeting tag), or (5'-promoter-targeting tag-5' flanking sequence-siRNA 1-loop sequence-compensation sequence-3' flanking sequence)-linker-(5'-promoter-targeting tag-5' flanking sequence-siRNA 2-loop sequence-compensation sequence-3' flanking sequence), or (5'-promoter-5' flanking sequence-siRNA 1-loop sequence-compensation sequence-3' flanking sequence)-linker-(5'-promoter-targeting tag-5' flanking sequence-siRNA 2-loop sequence-compensation sequence-3' flanking sequence), or (5'-promoter-targeting tag-5' flanking sequence-siRNA 1- siRNA 2- loop sequence-compensation sequence-3' flanking sequence), etc. Other situations can be deduced in this way and will not be repeated here. The above linker can be "sequence 1-sequence 2-sequence 3" or "sequence 4", and a bracket indicates a complete gene circuit.

Preferably, the above-mentioned RNA can also be obtained by ribose modification of the RNA sequence (siRNA, shRNA or miRNA), preferably 2' fluoropyrimidine modification. 2' Fluoropyrimidine modification is to replace the 2'-OH of the pyrimidine nucleotide of siRNA, shRNA or miRNA with 2'-F. 2'-F modification can make siRNA, shRNA or miRNA difficult to be recognized by RNase in the human body, thereby increasing the stability of RNA during transportation in vivo.

Specifically, exogenous plasmids are engulfed by the liver, with up to 99% of the exogenous plasmids entering the liver. Therefore, when the plasmids are used as carriers, they can be enriched in the liver tissue without specific design. Subsequently, the exogenous plasmids are opened, releasing RNA molecules (siRNA, shRNA, or miRNA). The liver tissue spontaneously encapsules the RNA molecules described above into the exosomes (self-assembly), allowing these exosomes then becoming a RNA delivery mechanism.

Preferably, in order to provide the RNA delivery mechanism (exosomes) with the ability of "precise guidance", we designed a targeting tag in the plasmid injected into the body, which will also be assembled into the exosomes by the liver tissue. Especially when certain specific targeting tags are selected, the targeting tags can be inserted on the surface of the exosomes, thus becoming a targeted structure that can guide the exosomes, which will greatly improve the accuracy of the RNA delivery mechanism described in the present invention. On the one hand, it can greatly reduce the amount of the exogenous plasmids required to be introduced, and on the other hand, it also greatly improves the efficiency of potential drug delivery..

Targeting tags are selected from one of the targeting peptides, proteins, or antibodies that have targeting functions. The selection of targeting tags requires creative labor. On the one hand, it is necessary to select available targeting tags based on the target tissue, and on the other hand, it is also necessary to ensure that the targeting tags can stably appear on the surface of the exosomes to achieve targeting functions. At present, targeting peptides that have been screened include but are not limited to RVG targeting peptide (with the nucleotide sequence as shown in SEQ ID No: 1), GE11 targeting peptide (with the nucleotide sequence as shown in SEQ ID No: 2), PTP targeting peptide (with the nucleotide sequence as shown in SEQ ID No: 3), TCP-1 targeting peptide (with the nucleotide sequence as shown in SEQ ID No: 4), or MSP targeting peptide (with the nucleotide sequence as shown in SEQ ID No: 5); targeting proteins include but are not limited to RVG-LAMP2B fusion protein (with the nucleotide sequence as shown in SEQ ID No: 6), GE11-LAMP2B fusion protein (with the nucleotide sequence as shown in SEQ ID No: 7), PTP-LAMP2B fusion protein (with the nucleotide sequence as shown in SEQ ID No: 8), TCP-1-LAMP2B fusion protein (with the nucleotide sequence as shown in SEQ ID No: 9), or MSP-LAMP2B fusion protein (with the nucleotide sequence as shown in SEQ ID No: 10).

Among them, RVG targeting peptide and RVG-LAMP2B fusion protein can precisely target brain tissue; GE11 targeting peptide and GE11-LAMP2B fusion protein can precisely target organs or tissues with high expression of EGFR, such as lung cancer tissue with EGFR mutation; PTP targeting peptide and PTP-LAMP2B fusion protein can precisely target the pancreas, especially the plectin-1 protein specifically expressed in human and mouse pancreatic cancer tissues; TCP-1 targeting peptide and TCP-1-LAMP2B fusion protein can precisely target the colon; and MSP targeting peptide and MSP-LAMP2B fusion protein can precisely target muscle tissue.

In practical applications, the targeting tag can be flexibly matched with various RNA fragments, and different targeting tags can play different roles with different RNA fragments. For example, RVG targeting peptide and RVG-LAMP2B fusion protein can be combined with siRNA of EGFR gene, siRNA of TNC gene or a combination of the two to treat glioblastoma, be combined with siRNA of PTP1B gene to treat obesity, be combined with siRNA of mHTT gene to treat Huntington's disease, or be combined with siRNA of LRRK2 gene to treat Parkinson's disease; GE11 targeting peptide and GE11-LAMP2B fusion protein can be combined with siRNA of EGFR gene to treat lung cancer and other diseases induced by high expression or mutation of EGFR gene; TCP-1 targeting peptide or TCP-1-LAMP2B fusion protein can be combined with siRNA of TNF-α gene, siRNA of integrin-α gene, siRNA of B7 gene or any combination of the above three to treat colitis or colon cancer.

In addition, in order to achieve precise delivery, we have experimented with various plasmid carrier loading schemes and obtained another optimized solution: the plasmid carrier can also be composed of multiple plasmids with different structures, one of which contains a promoter and a targeting tag, while the other plasmid contain a promoter and a RNA fragment. That is, the targeting tag and the RNA fragment are loaded into different plasmid vectors, and the two plasmid vectors are injected into the body, the targeting effect of which is not inferior to that of loading the same targeting tag and RNA fragment into one plasmid vector.

More preferably, when two different plasmid vectors are injected into the host body, the plasmid vector containing the RNA sequence can be injected first, and then the plasmid vector containing the targeting tag can be injected 1-2 hours later, so as to achieve better targeting effect.

The delivery systems described above can be used in mammals including humans.

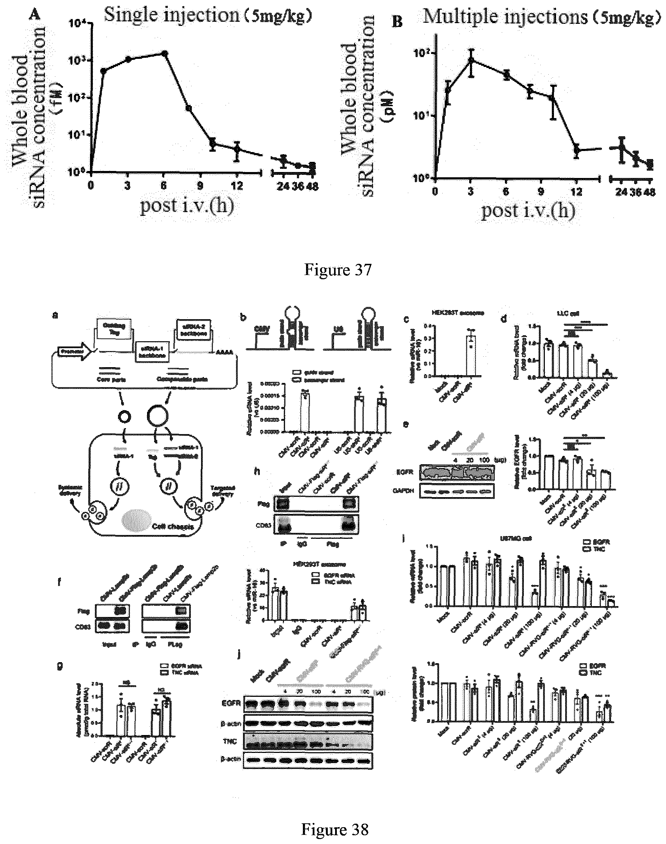

To verify the feasibility of the delivery system, the following experiments were conducted:
First, referring to Figure 38a, we rationally designed a basic genetic circuit architecture that allows the free combination of different functional modules. The core circuit, consisting of a promoter part and an siRNA-expressing part, was designed to produce and organize siRNAs as the payload for exosomes. Other composable parts (plug-ins) could then be integrated into the framework of the core circuit to achieve plug-and-play functionality. As an example, two types of composable parts were incorporated to optimize the siRNA effects: one modifying the membrane-anchored proteins of exosomes to enable tissue selectivity; the other enabling the co-expression of a second siRNA for the simultaneous inhibition of two molecular targets.

For the core circuit construct, we designed a scheme that encodes an optimized siRNA expression backbone part under the control of a promoter part to maximize guide strand expression while minimizing undesired passenger strand expression, see Figure 38a. Epidermal growth factor receptor (EGFR), one of the most potent oncogenes that is frequently mutated and highly expressed in a variety of human cancers (e.g., lung cancer and glioblastoma), was selected as the siRNA target. Human embryonic kidney 293T cells (HEK293T) and mouse hepatoma cells (Hepa 1-6) were chosen as the cell chassis for in vitro assembly of siRNA. To optimize the siRNA production efficiency, we compared two classic design strategies: one using the CMV promoter to express an miRNA precursor (pre-miRNA) and substituting the miRNA sequence with siRNA, and the other using the U6 promoter to express a short hairpin RNA (shRNA). We first examined a series of pre-miRNA structures and selected pre-miR-155 as the optimal backbone for the production of siRNA. Then, the siRNA production efficiency of a CMV-directed pre-miRNA encoding an EGFR siRNA (CMV-siR^{E}) and a U6-directed EGFR shRNA (U6-shR^{E}) was compared, see Figure 38b. Both strategies had similar efficiencies in driving the transcription of the guide strand of EGFR siRNA; however, compared with the shRNA approach, the pre-miRNA approach produced less or no passenger strand (the passenger strand is seemingly degraded during the biogenesis of mature guide strand), see Figure 38b. Therefore, the CMV-driven pre-miRNA design was chosen to avoid off-target effects.

Next, we examined whether the core circuit directs the loading of siRNA into exosomes. HEK293T cells were transfected with the control scrambled circuit (CMV-scrR) or CMV-siRE circuit, and the exosomes in cell culture medium were characterized. Nanoparticle tracking analysis (NTA) revealed that similar amounts of exosomes were secreted in each group with similar size and distribution, peaking at 128-131 nm. Transmission electron microscopy (TEM) confirmed that the purified exosomes exhibited a typical round-shaped vesicular morphology and had correct size. Moreover, enrichment of specific exosome markers (CD63, TSG101 and CD9) was only detected in purified exosomes but not in cell culture medium. These results demonstrate that transfection with circuits did not affect the size, structure or number of exosomes generated by HEK293T cells. Finally, a significant amount of EGFR siRNA was detected in exosomes derived from HEK293T and Hepa 1-6 cells transfected with the CMV-siR^{E} circuit, see Figure 38c. When these exosomes were incubated with mouse Lewis lung carcinoma (LLC) cells, a dose-dependent knockdown of EGFR expression was achieved, see Figure 38d and Figure 38e, suggesting that the exosomal siRNAs were biologically functional.

For the design of the targeting tag of the circuit, a sequence encoding a targeting tag fused to the N-terminus of Lamp2b (a canonical exosome membrane protein) was inserted downstream of the CMV promoter, see Figure 38a). This tag was anchored to the exosome surface via Lamp2b, thus guiding the delivery of the complex exosome to the desired tissue. Specifically, a central nervous system-targeting RVG peptide was chosen as the tag to direct exosomes to the brain (RVG has been shown to facilitate transcytosis of exosomes across the blood-brain barrier and into neuronal cells). The efficiency of the promoters in initiating the expression of the RVG-Lamp2b fusion protein was first assessed. Experiments have shown that the CMV promoter has a certain effect in generating RVG-Lamp2b mRNA and marker protein eGFP in HEK293T cells, whereas the U6 promoter was not effective, confirming the advantage of the CMV promoter for linking each part of the circuit. The correct display of the targeting tag on the exosome surface was then verified using immunoprecipitation. A Flag epitope tag was used to replace RVG because of the lack of an anti-RVG antibody. After transfection of HEK293T and Hepa 1-6 cells with a CMV-directed Flag-Lamp2b circuit, intact exosomes were successfully immunoprecipitated with anti-Flag beads, see Figure 38f, demonstrating the precise localization of the targeting tag. For the design of additional siRNA-expressing parts, tenascin-C (TNC), a critical oncogene associated with many cancers, especially glioblastoma, was selected as the second siRNA target. TNC siRNA was also embedded in the pre-miR-155 backbone and inserted downstream of the EGFR siRNA, see Figure 38. Regardless of the individual (CMV-siR^{E} or CMV-siR^{T}) or tandem (CMV-siR^{E}) transcription, equal amounts of EGFR and TNC siRNA were detected, see Figure 38g. Overall, these results show that both the siRNA and targeting tag can play their respective roles in the circuit.

Next, we examined whether the circuit enables the self-assembly of siRNAs into exosomes in vitro. HEK293T cells were transfected with a CMV-directed circuit encoding both EGFR and TNC siRNAs along with an RVG tag (CMV-RVG-siR^{E}). The results showed that exosomes derived from the cell culture medium displayed typical morphology and size distribution, indicating that modification with the composable-core circuit did not alter the physical properties of exosomes. Moreover, a complete circuit encoding both EGFR and TNC siRNAs and a targeting tag (CMV-Flag-siR^{E}) was constructed. Exosomes generated from the transfected HEK293T and hep 1-6 cells were successfully immunoprecipitated, and both EGFR and TNC siRNAs were abundantly present in the immunoprecipitated exosomes, see Figure 38h.

Furthermore, AGO2 is widely expressed in organisms and is a core component of the RNA-induced silencing complex. It has endoribonuclease activity and can inhibit the expression of target genes by promoting the maturation of siRNA and regulating its biosynthesis and function.

Since siRNA processing is dependent on Argonaute 2 (AGO2) and the proper loading of siRNA into AGO2 is expected to enhance the on-target effects of siRNA, another immunoprecipitation experiment was performed to assess the association of AGO2 with siRNAs in exosomes. Experiments demonstrated that EGFR and TNC siRNAs were readily detected in the exosomes precipitated with anti-AGO2 antibody, suggesting that our design guarantees the loading of siRNA into the RNA-induced silencing complex (RISC) and facilitates the efficient transport of AGO2-bound siRNA into exosomes. Finally, to investigate whether the in vitro assembled siRNAs are functional, exosomes derived from HEK293T cells transfected with the CMV-RVG-siR^{E+T} circuit were incubated with the U87MG glioblastoma cells. A dose-dependent downregulation of EGFR and TNC expression in U87MG cells was achieved, see Figure 38i and Figure 38j. Moreover, the RVG tag on exosome surface did not affect the silencing efficacy of EGFR and TNC siRNAs on their targets. These results establish the circuit as an organic combination of multiple composable parts that direct the self-assembly and release of siRNAs in vitro.

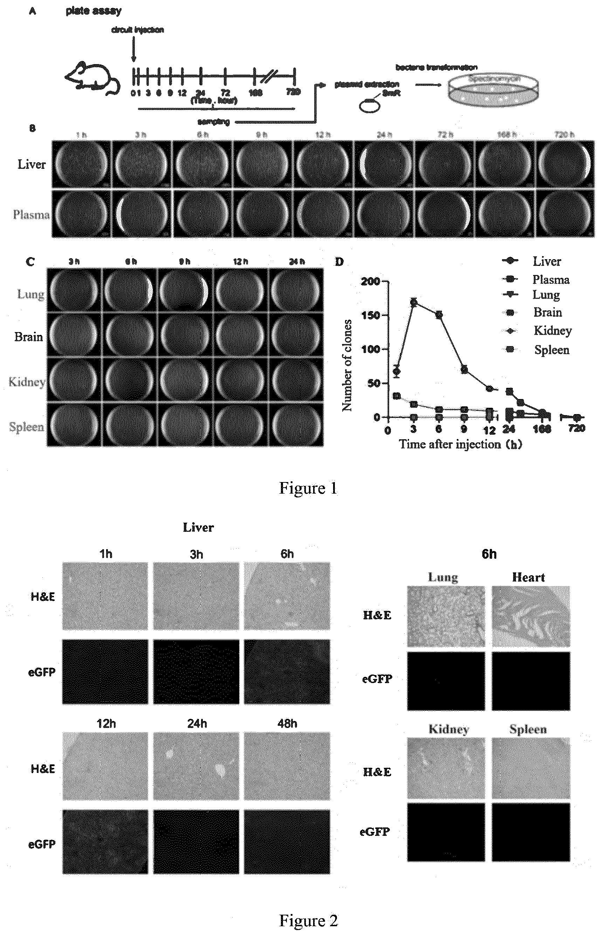

In order to understand the distribution of the plasmid in the body, we conducted a plate test on mice. As shown in Figure 1A, after the plasmid was injected into the mice, samples were taken at various time points (1h, 3h, 6h, 9h, 12h, 24h, 72h, 168h, 720h). The plasmid extracted with spectinomycin was used for transformation, and the number of clones in the liver, plasma, lung, brain, kidney, and spleen was observed. The results are shown in Figure 1B, Figure 1C, and Figure 1D. It can be seen that the plasmid was mostly distributed in the mouse liver, reached its peak about 3 h after injection, and was basically metabolized 12 h after injection.

CMV eGFP siR^{E} circuit co-expressing eGFP protein and EGFR siRNA was injected intravenously into C57BL/6J mice. The results are shown in Figure 2 that the eGFP fluorescence in the mouse liver gradually increased over time, reached a peak at about 12 h, and decreased to the background level at 48 h. No obvious eGFP signal was seen in other tissues.

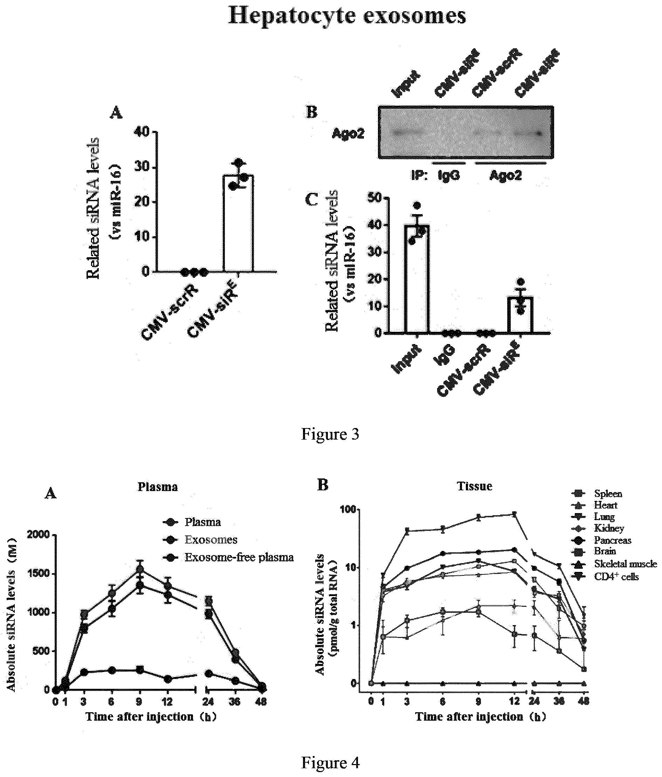

The control plasmid (CMV-scrR) and the plasmid expressing EGFR siRNA (CMV-siR^{E}) were injected into the mice respectively, and an in vitro model of mouse hepatocytes was established. The related siRNA levels in the hepatocyte exosomes of mice injected with CMV-scrR and CMV-siR^{E} were respectively detected. The results are shown in Figure 3A. It can be seen that siRNA expression was present in the hepatocyte exosomes of mice injected with CMV-siR^{E}.

It is generally believed that binding to Ago2 protein is a necessary condition for siRNA to function, meaning that siRNA in exosomes can bind to Ago2 protein. Therefore, we performed Ago2 immunoprecipitation experiments, and the results are shown in Figure 3B and Figure 3C. Wherein, Input represents a sample in which exosomes were directly lysed and detected without immunoprecipitation, serving as a positive control.

After intravenous injection of the plasmid into mice, the distribution of mature siRNA in different tissues is shown in Figure 4. It can be seen from Figure 4A that the level of EGFR-siRNA in plasma, exosomes, and plasma without exosomes varied in a time-dependent manner; as can be seen from Figure 4B, the accumulation of mouse EGFR-siRNA in the liver, lung, pancreas, spleen, and kidney was time-dependent.

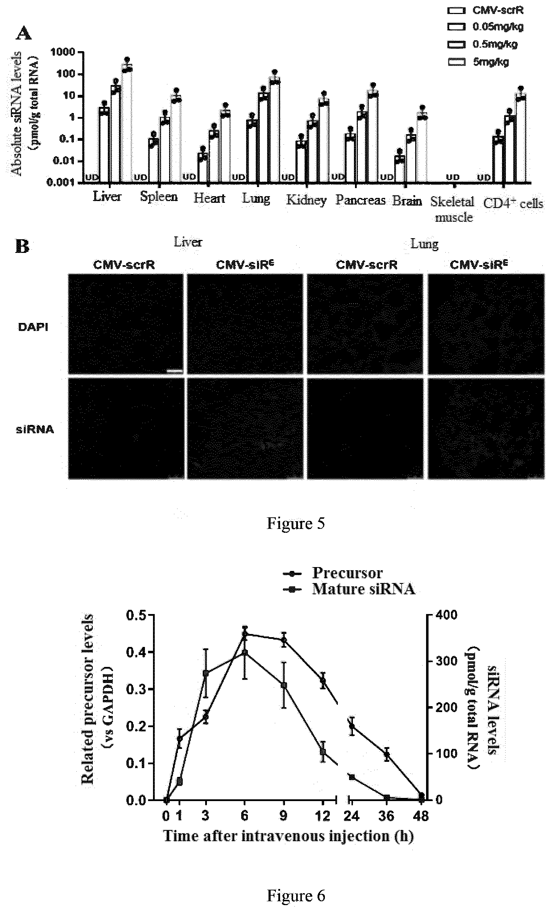

Mice were injected with control plasmid (CMV-scrR), 0.05 mg/kg CMV-siR^{E} plasmid, 0.5 mg/kg CMV-siR^{E} plasmid, and 5 mg/kg CMV-siR^{E} plasmid, respectively, and the level of absolute siRNA (EGFR siRNA) was detected in the mouse liver, spleen, heart, lung, kidney, pancreas, brain, skeletal muscle, and CD4⁺ cells. The results are shown in Figure 5A. It can be seen that there was no expression of siRNA in the tissues of mice injected with control plasmid, and in the tissues of mice injected with CMV-siR^{E} plasmid, the level of siRNA expression was positively correlated with the concentration of CMV-siR^{E} plasmid. As shown in Figure 5B, fluorescence in situ hybridization assay (FISH) also confirmed that the level of siRNA expression was positively correlated with the concentration of CMV-siR^{E} plasmid, indicating that the tissue distribution of EGFR siRNA was dose-dependent.

After the plasmid enters the body, it will express the precursor and then process it into a mature body (siRNA). Therefore, we detected the metabolism of the precursor and mature body (siRNA) in the liver of mice injected with the plasmid, and the result is shown in Figure 6. It can be seen that the expression levels of precursor and mature body (siRNA) in the mouse liver reached the peak at 6 h after the injection of the plasmid; 36 h after plasmid injection, the metabolism of the mature body (siRNA) was completed in the mouse liver; and 48 h after the plasmid injection, the metabolism of the precursor was completed in the mouse liver.

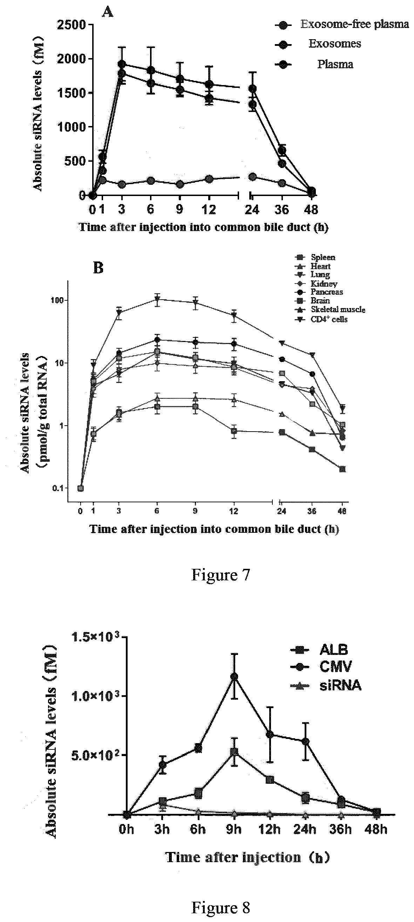

After injecting exogenous siRNA into the common bile duct of mice, the levels of absolute siRNA in exosome-free plasma, exosomes, and plasma were detected respectively. The results are shown in Figure 7A. After injecting exogenous siRNA into the common bile duct of mice, the levels of siRNA in the spleen, heart, lung, kidney, pancreas, brain, skeletal muscle, and CD4⁺ cells of mice were detected respectively. The results are shown in Figure 7B. These two figures reflect that the kinetics of siRNA in different tissues was almost the same, and the distribution of siRNA in different tissues was significantly different.

Mice were intravenously injected with siRNA with albumin ALB as promoter, siRNA with CMV as promoter, and siRNA without any promoter respectively. The absolute siRNA levels in mice were detected at 0h, 3h, 6h, 9h, 12h, 24h, 36h, and 48h after injection, and the results are shown in Figure 8. It can be seen that the level of siRNA using CMV as the promoter was the highest in mice, that is, using CMV as the promoter had the best effect.

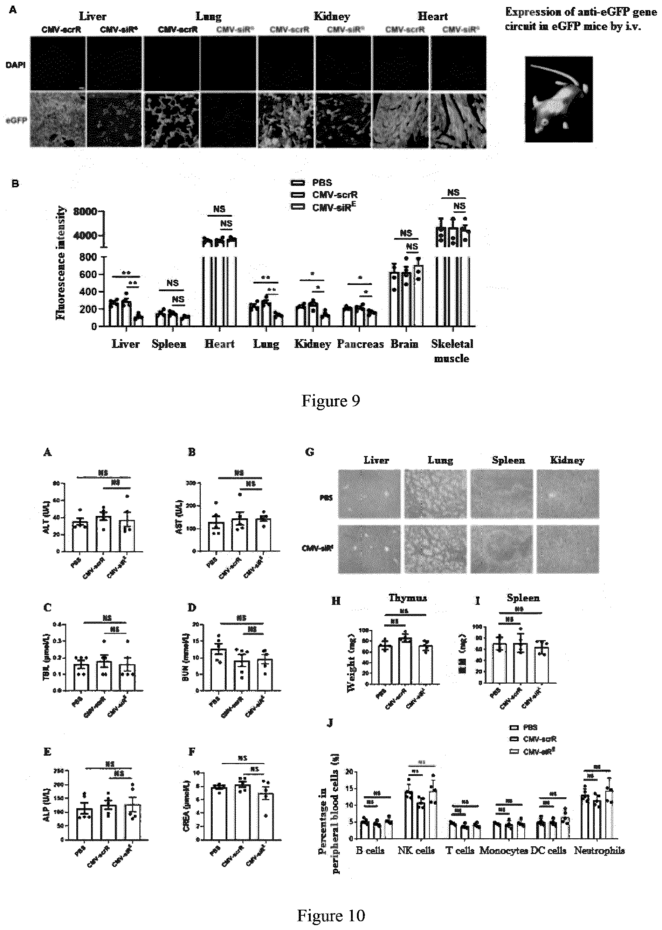

We used fluorescence experiments to observe the inhibition of eGFP levels in mice by self-assembled eGFP siRNA. The process was as follows: eGFP transgenic mice were intravenously injected with PBS, or 5 mg/kg CMV-siR^{G} or CMV-RVG-siR^{G} plasmid, and treated for 24 h. The mice were then sacrificed, and their eGFP fluorescence levels were detected in frozen sections. Figure 9A shows a representative fluorescence microscope image, in which green indicates positive eGFP signals and blue indicates DAPI-stained nuclei, with a scale bar of 100 µm. It can be seen that the inhibitory effect of CMV-RVG-siR^{G} plasmid on mouse eGFP was more obvious. eGFP transgenic mice were intravenously injected with PBS, or CMV-scrR or CMV-siR^{E} plasmid, and treated for 24 h. The mice were then sacrificed, and their eGFP fluorescence levels were detected in frozen sections. Figure 9B is a column comparison chart of the fluorescence intensity of the heart, lung, kidney, pancreas, brain, and skeletal muscle of mice injected with PBS, CMV-siR^{E}, and CMV-RVG-siR^{E}. It can be seen that the fluorescence intensity was more obvious in the liver, spleen, lung, and kidney of mice.

The alanine aminotransferase (ALT), aspartate aminotransferase (AST), total bilirubin (TBIL), blood urea nitrogen (BUN), serum alkaline phosphatase (ALP), creatinine (CREA) contents, thymus weight, spleen weight, and percentage in peripheral blood cells were detected in mice injected with PBS, CMV-scrR, and CMV-siR^{E} respectively. The results are shown in Figure 10. Figures 10A-F show the comparison of alanine aminotransferase, aspartate aminotransferase, total bilirubin, blood urea nitrogen, serum alkaline phosphatase, and creatinine contents in mice injected with PBS, CMV-scrR, and CMV-siR^{E}; Figure 10G show the comparison of mouse liver, lung, spleen, and kidney tissues, Figures 10H-I show the comparison of mouse thymus and spleen tissues, and Figure 10J shows the comparison of percentage in mouse peripheral blood cells.

The results showed that mice injected with PBS, CMV-scrR, and CMV-siR^{E} had almost the same ALT, AST contents, thymus weight, spleen weight, and percentage in peripheral blood cells. Compared with mice injected with PBS, mice injected with CMV-siRE also had no tissue damage in the liver, lung, spleen, and kidney.

Therefore, the RNA delivery system provided in this example uses a plasmid as a carrier, and as a mature injection, the plasmid has safety and reliability that have been fully verified, and has a very good druggability. The RNA sequence that exerts the final effect is encapsulated and transported by endogenous exosomes, with no immune responses, and there is no need to verify the safety of the exosomes. The delivery system can deliver all kinds of small molecule RNAs and has strong versatility. Moreover, the preparation of plasmids is much cheaper than the preparation of exosomes, proteins, polypeptides and the like, with good economy. The RNA delivery system provided in this example can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

### Example 2

On the basis of Example 1, an medicament is provided in this example. The medicament comprises an plasmid carrying a RNA fragment required to be delivered, the plasmid can be enriched in an organ tissue of a host, and spontaneously forms a complex structure containing the RNA fragment in the organ tissue of the host, the complex structure can enter and be combined with a target tissue to deliver the RNA fragment into the target tissue. Optionally, the RNA fragment comprises one, two or more specific RNA sequences with medical significance, which are siRNA, shRNA, or miRNA with medical significance.

Optionally, the RNA fragment comprises one, two or more specific RNA sequences with medical significance, which are siRNA, shRNA, or miRNA with medical significance.

In Figures 39-41, the six types of RNAs are: siRE (targeted for EGFR gene), siRT (targeted for TNC gene), shRE (targeted for EGFR gene), shRT (targeted for TNC gene), miR-7 (targeted for EGFR gene), and miR-133b (targeted for EGFR gene), respectively. Figure 39 shows the enrichment effect of plasmids containing 6 different RNAs in plasma, the detection of siRNA in the exosomes and corresponding gene expression levels; Figure 40 shows the enrichment effect of four groups of plasmids consisted of any two RNA sequences of the six RNAs provided above in plasma, the detection of siRNA in the exosomes and corresponding gene expression levels; Figure 41 shows the enrichment effect of three plasmids consisted of any three RNA sequences of the six RNAs provided above in plasma, the detection of siRNA in the exosomes and corresponding gene expression levels.

The RNA sequences are shown specifically in Table 5.

| **Name** | **Sequence** |
|---|---|
| siRE precursor sequence | ATACCTATTCCGTTACACACTGTTTTGGCCACTGACTGA CAGTGTGTAGGAATAGGTAT |
| siRT precursor sequence | CACACAAGCCATCTACACATGGTTTTGGCCACTGACTG ACCATGTGTATGGCTTGTGTG |
| shRE precursor sequence | ATACCTATTCCGTTACACACTGTTTTGGCCACTGACTGA CAGTGTGTAACGGAATAGGTAT |
| shRT precursor sequence | CACACAAGCCATCTACACATGGTTTTGGCCACTGACTG ACCATGTGTAGATGGCTTGTGTG |
| miR-7 | hsa-miR-7 |
| miR-133b | hsa-miR-133b |

Optionally, the plasmid further comprises a promoter and a targeting tag, the targeting tag can form a targeting structure of the complex structure in the organ tissue of the host, the targeting structure is located on the surface of the complex structure, and the complex structure can search for and be combined with the target tissue through the targeting structure to deliver the RNA fragment into the target tissue.

The explanations of the above plasmids, RNA fragments, targeting tags, etc. in this Example can all be refered to Example 1, and will not be repeated here.

The medicament can enter the human body by oral administration, inhalation, subcutaneous injection, intramuscular injection or intravenous injection, and then be delivered to the target tissue through the RNA delivery system described in Example 1 to exert a therapeutic effect.

The medicament can be used to treat cancer, pulmonary fibrosis, colitis, obesity, cardiovascular disease caused by obesity, type 2 diabetes, Huntington's disease, Parkinson's disease, myasthenia gravis, Alzheimer's disease or graft-versus-host disease.

The medicament in this example may also comprise a pharmaceutically acceptable carrier, which includes but is not limited to diluents, buffers, emulsions, encapsulating agents, excipients, fillers, adhesives, sprays, transdermal absorption agents, wetting agents, disintegrants, absorption accelerators, surfactants, colorants, flavoring agents, adjuvants, desiccants, adsorption carriers, etc.

The dosage form of the medicament provided in this example may be tablets, capsules, powders, granules, pills, suppositories, ointments, solutions, suspensions, lotions, gels, pastes, etc..

The medicament provided in this example uses a plasmid as a carrier, and as a mature injection, the plasmid has safety and reliability that have been fully verified, and has a very good druggability. The RNA sequence that exerts the final effect is encapsulated and transported by endogenous exosomes, with no immune responses, and there is no need to verify the safety of the exosomes. The medicament can deliver all kinds of small molecule RNAs and has strong versatility. Moreover, the preparation of plasmids is much cheaper than the preparation of exosomes, proteins, polypeptides and the like, with good economy. The medicament provided in this application can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

### Example 3

On the basis of Examples 1 or 2, use of an RNA delivery system in the manufacture of a medicament for treating a disease is provided in this example. The medicament is used for treating lung cancer. Here, it will be specifically described by the following experiments.

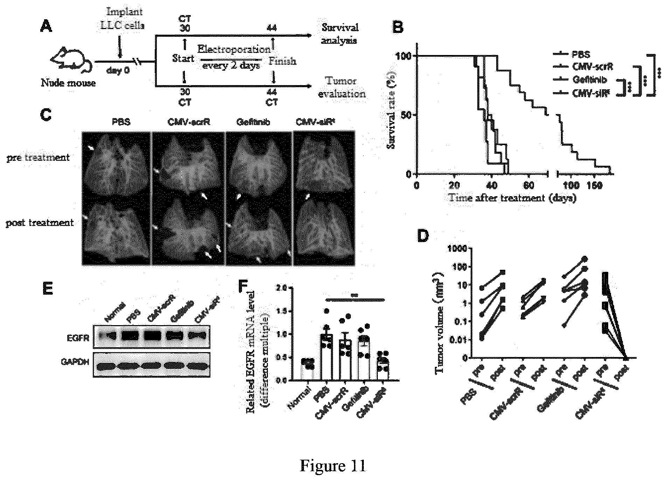

As shown in Figure 11A, mice were selected, and injected with mouse lung cancer cells (LLC cells), followed by PBS buffer/CMV-scrR/gefitinib/CMV-siR^{E} every two days for treatment. Survival analysis and tumor evaluation were performed on the mice. Treatment started on the 30th day and ended on the 44th day.

As shown in Figure 11B, the horizontal axis represents time and the vertical axis represents survival rate. From this figure, it can be seen that mice injected with CMV-siR^{E} had the highest survival rate.

As shown in Figure 11C, the figure shows the 3D modeling of mouse lung tissue according to the CT images of mice injected with PBS buffer/CMV-scrR/gefitinib/CMV-siR^{E} before and after treatment. It can be seen that the tumors of mice injected with CMV-siR^{E} were significantly reduced.

As shown in Figure 11D, the figure shows the comparison of the tumor volume (mm³) of mice injected with PBS buffer/CMV-scrR/gefitinib/CMV-siR^{E} before and after treatment. It can be seen that the tumor volume of mice injected with CMV-siR^{E} was significantly reduced, while the tumor volume of mice injected with PBS buffer/CMV scrR/gefitinib not only failed to decrease, but also increased to varying degrees.

As shown in Figure 11E, the figure shows the comparison of western blot results of normal mice and mice injected with PBS buffer/CMV-scrR/gefitinib/CMV-siR^{E}. It can be seen that mice injected with PBS buffer/CMV scrR/gefitinib had significantly higher levels of the EGFR gene.

As shown in Figure 11F, the figure shows the comparison of the levels of related EGFR miRNA in normal mice and mice injected with PBS buffer/CMV-scrR/gefitinib/CMV-siR^{E}. It can be seen that mice injected with PBS buffer/CMV-scrR/gefitinib had relatively higher levels of related EGFR miRNA.

In summary, CMV-siR^{E} had a significant therapeutic effect on EGFR-mutated lung cancer tumors.

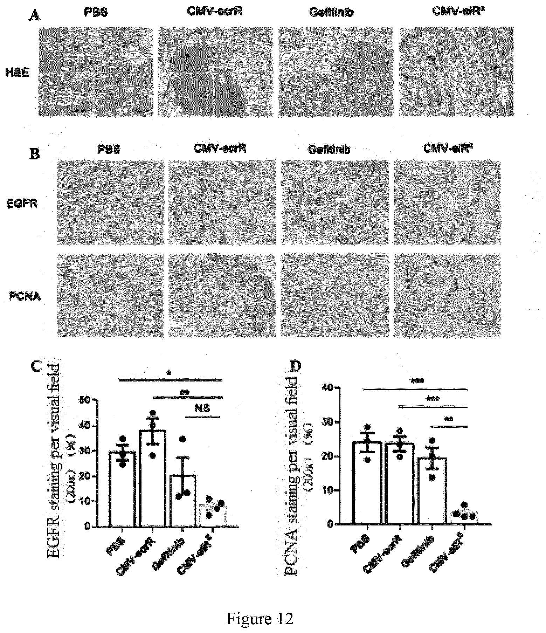

HE staining and immunohistochemical staining were performed on mice injected with PBS buffer/CMV-scrR/gefitinib/CMV-siR^{E} respectively. The results are shown in Figure 12A and Figure 12B. EGFR had more expression in mice injected with PBS buffer/CMV-scrR/gefitinib. The staining areas of EGFR and PCNA in mice were counted. The results are shown in Figure 12C and Figure 12D. It can be seen that the staining areas of EGFR and PCNA in mice injected with CMV siR^{E} were the smallest, proving it had the best therapeutic effect on EGFR-mutated lung cancer tumors.

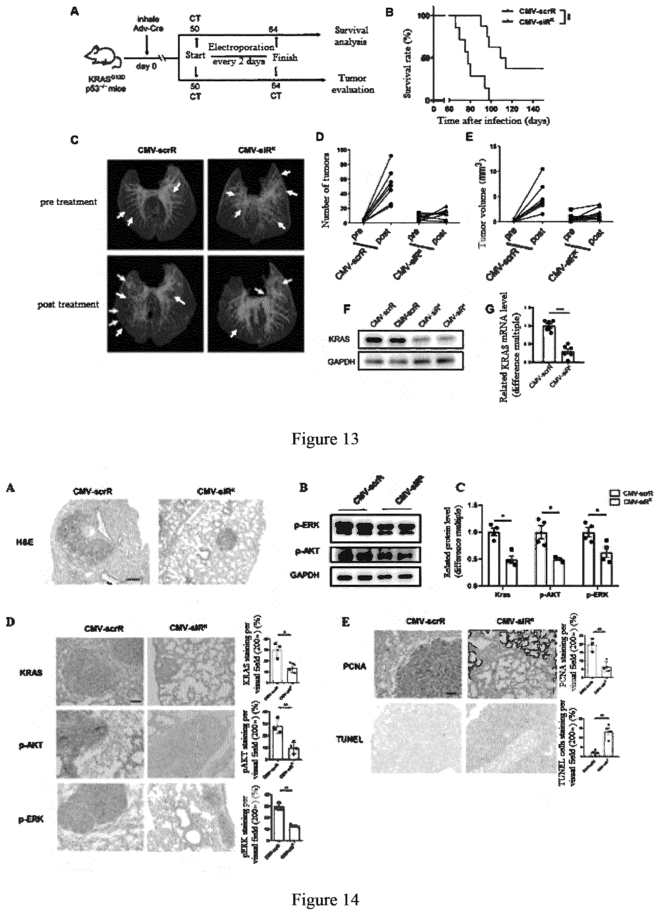

As shown in Figure 13A, KRAS^{G12D} p53^{-/-} mice were selected and inhaled with Adv-Cre. From Day 50 to Day 64 after inhalation, mice were injected with PBS buffer/CMV-scrR/gefitinib/CMV-siR^{E} every two days for treatment, and survival analysis and tumor evaluation of the mice were performed.

As shown in Figure 13B, the horizontal axis represents the time after infection, and the vertical axis represents the survival rate. It can be seen from this figure that the survival rate of mice injected with CMV-siR^{K} was higher.

As shown in Figure 13C, the figure shows the 3D modeling of the mouse lung tissue according to the CT images of mice injected with CMV-scrR/CMV-siR^{K} before and after treatment. It can be seen that the injection of CMV-siR^{K} can significantly inhibit the growth of lung cancer tumors.

As shown in Figure 13D, the figure shows the comparison of the number of tumors in mice injected with CMV-scrR/CMV-siR^{K} before and after treatment. It can be seen that the number of tumors in mice injected with CMV-siR^{K} increased significantly less.

As shown in Figure 13E, the figure shows the comparison of tumor volume (mm³) of mice injected with CMV-scrR/CMV-siR^{K} before and after treatment. It can be seen that the tumor volume of mice injected with CMV-siR^{K} grew slowly, while the tumor volume of mice injected with CMV-scrR increased significantly.

As shown in Figure 13F, the figure shows the comparison of western blot results of mice injected with CMV-scrR/CMV-siR^{K}. It can be seen that the level of KRAS gene in mice injected with CMV-scrR was significantly higher.

As shown in Figure 13G, the figure shows the comparison of the levels of related KRAS mRNA in mice injected with CMV-scrR/CMV-siR^{K}. It can be seen that the levels of related KRAS mRNA in mice injected with CMV-scrR were relatively high.

In summary, CMV-siR^{K} had a significant therapeutic effect on KRAS-mutated lung cancer tumors.

HE staining and immunohistochemical staining were performed on mice injected with CMV-scrR/CMV-siR^{K}. The results are shown in Figure 14A, Figure 14D, and Figure 14E. It can be seen that mice injected with CMV-scrR had more expression of KRAS, p-AKT, and p-ERK, and a higher staining percentage. Western blot was used to detect the expression levels of related proteins in mice. As shown in Figure 14B and Figure 14C, mice injected with CMV-scrR had more expression of related proteins. This also shows that CMV-siR^{K} had a significant inhibitory effect on KRAS mutated lung cancer tumors.

### Example 4

On the basis of Example 1 or 2, use of an RNA delivery system in the manufacture of a medicament for treating a disease is provided in this example. The medicament is used for treating kidney cancer.

This is illustrated in detail through the following experiments.

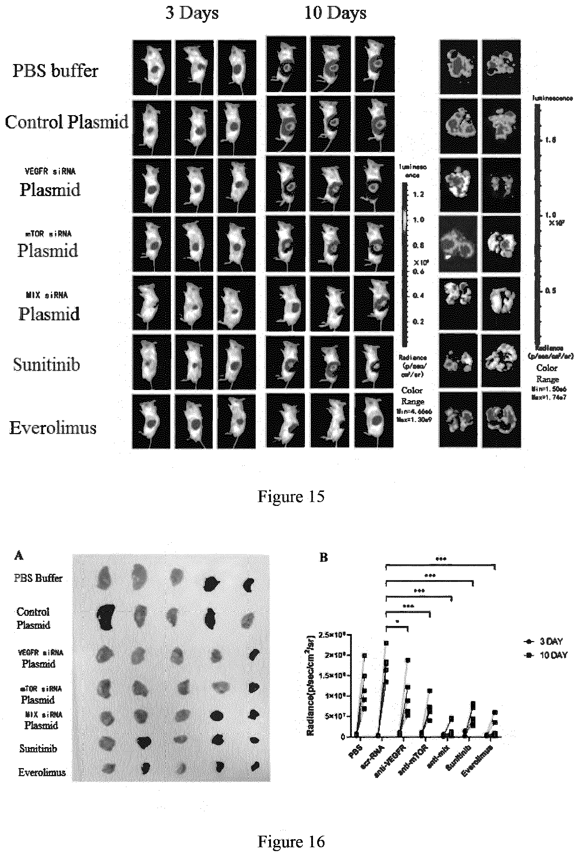

Different mice were injected with PBS buffer/control plasmid/VEGFR siRNA plasmid/mTOR siRNA plasmid/MIX siRNA plasmid (combined use of VEGFR siRNA and mTOR siRNA)/sunitinib/everolimus. The development of kidney cancer tumors in mice was observed, and the results are shown in Figure 15 and Figure 16. It can be seen that the development of kidney cancer in mice injected with MIX siRNA plasmid was most significantly inhibited, while the development of kidney cancer in mice injected with PBS buffer/control plasmid was more rapid.

In summary, the combined use of VEGFR siRNA and mTOR siRNA had a significant therapeutic effect on kidney cancer tumors.

### Example 5

On the basis of Example 1 or 2, use of an RNA delivery system in the manufacture of a medicament for treating a disease is provided in this example. The medicament is used for treating colitis. This example will specifically illustrate the effect of the RNA delivery system in the treatment of colitis through the following two experiments.

In the first experiment, we set up 3 experimental groups and 3 control groups. The experimental groups were anti-TNF-α (0.5) group, anti-TNF-α (5) group and anti-TNF-α (20) group; and the control groups were mock group, scr-RNA group and IFX group.

Among them, the anti-TNF-α (0.5) group, anti-TNF-α (5) group, and anti-TNF-α (20) group respectively used plasmids to encapsulate the TNF-α siRNA system (CMV-siR^{TNF-α}). 0.5 µL, 5 µL, and 20 µL of CMV-siR^{TNF-α} solutions were injected into the mice through the tail vein.

The mock group was the negative control group, and the scr-RNA group and IFX group were injected with scr-RNA plasmid and IFX (infliximab) through the tail vein of mice respectively.

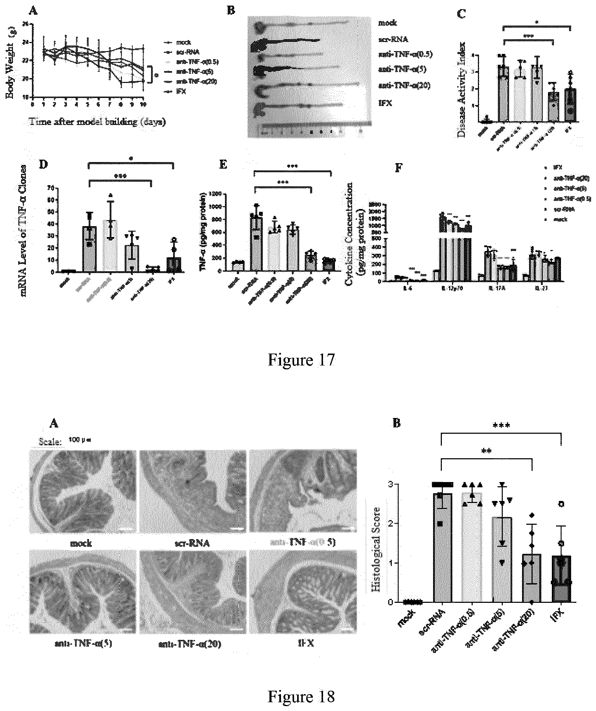

DSS-induced chronic colitis model was then constructed, during which weights were recorded every day. The results are shown in Figure 17A. It can be seen that mice in the scr-RNA group experienced the most rapid weight loss, while mice in the anti-TNF-α (0.5) group, anti-TNF-α (5) group, and anti-TNF-α (20) group experienced a slower weight loss, and the higher the dose of TNF-α siRNA solution, the slower the weight loss of the mice, showing that the plasmid-encapsulated TNF-α siRNA system can reduce weight loss in colitis mice.

After the model was constructed, the in vivo expression of the plasmid system was monitored by small animal in vivo imaging, and then the mice were sacrificed for observation of the colon. The results are shown in Figure 17B. It can be seen that mice in the scr-RNA group had the shortest colon length, while mice in the anti-TNF-α (0.5) group, anti-TNF-α (5) group, and anti-TNF-α (20) group had relatively longer colon length, and the higher the dose of TNF-α siRNA injected, the longer the mouse colon length, showing that the plasmid-encapsulated TNF-α siRNA system can improve the shortening of colon length caused by chronic inflammation to varying degrees.

The disease activity index of the mice was evaluated, and the results are shown in Figure 17C. It can be seen that mice in the scr-RNA group, anti-TNF-α (0.5) group, and anti-TNF-α (5) group had a higher disease activity index, while mice in the anti-TNF-α (20) group and IFX group had a smaller disease activity index.

TNF-α mRNA was detected in mouse colon, and the results are shown in Figure 17D. It can be seen that the CMV-siR^{TNF-α} system can reduce the expression and secretion of colon TNF-α. TNF-α was detected in mouse colon, and the results are shown in Figure 17E. It can be seen that the AAV system can produce a certain amount of TNF-α. The pro-inflammatory factors IL-6, IL-12p70, IL-17A, and IL-23 in the colon were detected, and the results are shown in Figure 17F. It can be seen that the secretion of inflammatory factors in the high-dose group was lower than that in the control group as a whole.

HE staining of mouse colon sections and pathological score statistics were performed, and the results are shown in Figure 18A and Figure 18B. It can be seen that mice in the anti-TNF-α (0.5) group, anti-TNF-α (5) group, anti-TNF-α (20) group, especially the anti-TNF-α (20) group, had higher colon mucosal integrity, shallower immune cell infiltration, and significantly less colonic crypt abscesses and colon congestion and bleeding compared to the control groups.

The above experiments can prove that the use of plasmid-encapsulated TNF-α siRNA system treatment was more effective than or at least as effective as IFX in improving the manifestations of colitis.

In the second experiment, we set up 4 experimental groups and 3 control groups. The experimental groups were anti-TNF-α group, anti-integrin-α group, anti-B7 group, and anti-mix group. The control groups were mock group, PBS group, and scr-RNA group.

The anti-TNF-α group, anti-integrin-α group, anti-B7 group, and anti-mix group used plasmids to encapsulate the TNF-α siRNA system (CMV-siR^{TNF-α}), integrin-α siRNA system (CMV-siR^{integrin-α}), B7 siRNA system (CMV-siR^{B7}), mix siRNA (CMV-siR^{mix}, i.e., CMV-siR^{TNF-α+integrin-α+B7}) system. 20 µL was injected into the mice through tail vein, and the expression of the system was monitored in vivo in small animals. It can be seen that the above system was stably expressed in vivo, especially in the liver.

The mock group was the negative control group, and mice in the scr-RNA group and PBS group were injected with scr-RNA plasmid and PBS solution (phosphate buffered saline solution) through the tail vein, respectively.

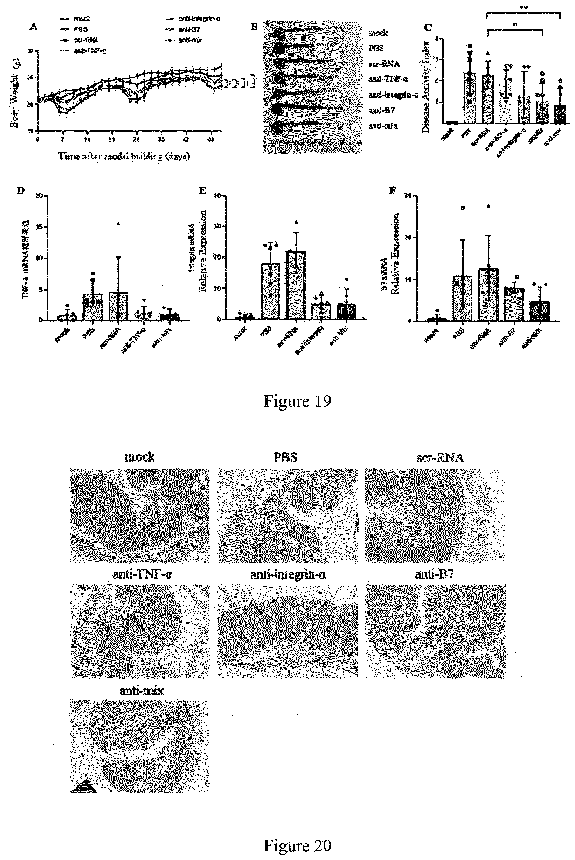

DSS-induced chronic colitis model was then constructed, during which weights were recorded every day. The results are shown in Figure 19A. It can be seen that the weight gain of mice in the anti-mix group was the most stable, indicating that the plasmid-encapsulated CMV siR^{TNF-α+integrin-α+B7} system can significantly reduce the weight loss of mice with chronic colitis. The mice in the anti-TNF-α group, anti-integrin-α group, and anti-B7 group also had a significantly faster weight recovery than the scr-RNA group and PBS group during the inflammation remission period.

After the model construction was completed, the in vivo expression of the plasmid system was monitored by small animal in vivo imaging, and then the mice were sacrificed for observation of the colon. The results are shown in Figure 19B. It can be seen that the redness and swelling of the colon of mice in the four experimental groups were alleviated to varying degrees. The shortening of colon length caused by chronic inflammation also improved to varying degrees.

The disease activity index of mice was evaluated, and the results are shown in Figure 19C. It can be seen that the disease activity index of mice in the scr-RNA group and PBS group was higher, while the disease activity index of mice in the anti-TNF-α group, anti-integrin-α group, anti-B7 group, and anti-mix group decreased in sequence.

TNF-α mRNA, integrin mRNA and B7 mRNA were detected in mouse plasma, liver and colon. The results are shown in Figure 19D-Figure 19F. It can be seen that the system generated a certain amount of stably expressed RNA in plasma, liver and colon, and the system significantly reduced expression of TNF-α, integrin and B7 mRNA in colon.

HE staining was performed on mouse colon sections. The results are shown in Figure 20. It can be seen that mice in the four experimental groups, especially mice in the anti-mix group, had higher colon mucosal integrity, shallower immune cell infiltration, and significantly less colonic crypt abscesses and colon congestion and bleeding compared to the control groups.

The above experiments show that the use of the plasmid with high affinity for the liver to encapsulate the CMV-siR^{TNF-α+integrin-α+B7} circuit can achieve long-term expression of TNF-α mRNA, B7 mRNA and integrin mRNA and multiple target gene silencing, and can significantly alleviate the degree of colonic inflammation, with great drug potential and clinical research value.

### Example 6

On the basis of Example 1 or 2, use of an RNA delivery system in the manufacture of a medicament for treating a disease is provided in this example. The medicament is used for treating pulmonary fibrosis. This example will specifically illustrate the use of the RNA delivery system in the treatment of pulmonary fibrosis through the following experiments.

In this example, 8 experimental groups and 3 control groups were set up. The experimental groups were Anti-miR-21 (1 mg/kg) group, Anti-miR-21 (5 mg/kg) group, Anti-miR-21 (10 mg/kg) group, TGF-β1 siRNA (1 mg/kg) group, TGF-β1 siRNA (5 mg/kg) group, TGF-β1 siRNA (10 mg/kg) group, Anti-miR-21+TGF-β1 siRNA (10 mg/kg) group, Pirfenidone (300 mg/kg) group; and the control group were Normal group, PBS group and scrRNA group.

Among them, mice with pulmonary fibrosis in the Anti-miR-21 (1 mg/kg) group, Anti-miR-21 (5 mg/kg) group, and Anti-miR-21 (10 mg/kg) group were injected into the tail vein with 1 mg/kg, 5 mg/kg, 10 mg/kg of the plasmid of miR-21 siRNA; mice with pulmonary fibrosis in TGF-β1 siRNA (1 mg/kg) group, TGF-β1 siRNA (5 mg/kg) group, TGF-β1 siRNA (10 mg/kg) group were injected into the tail vein with 1 mg/kg, 5 mg/kg, 10 mg/kg of TGF-β1 siRNA plasmid; mice with pulmonary fibrosis in Anti-miR-21+TGF-β1 siRNA (10 mg/kg) group were injected into the tail vein with 10 mg/kg Anti-miR-21 and TGF-β1 siRNA plasmids; and mice with pulmonary fibrosis in Pirfenidone (300 mg/kg) group was injected into the tail vein with 300 mg/kg Pirfenidone. The Normal group was the normal control group, and mice with pulmonary fibrosis in the PBS group and the scrRNA group were injected into the tail vein with PBS solution and control plasmid, respectively.

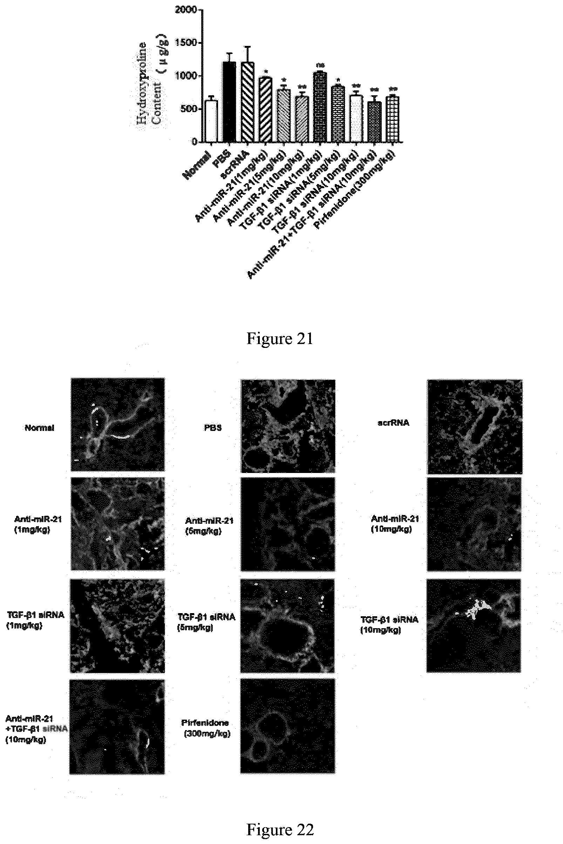

The hydroxyproline content of mice in each group was detected respectively, and the results are shown in Figure 21. Hydroxyproline is the main component of collagen, and its content reflects the degree of pulmonary fibrosis. As can be seen from Figure 21, mice in the Anti-miR-21 (5 mg/kg) group, Anti-miR-21 (10 mg/kg) group, TGF-β1 siRNA (10 mg/kg) group, and Anti-miR-21+TGF-β1 siRNA (10 mg/kg) group had relatively low hydroxyproline content, indicating that their pulmonary fibrosis was inhibited.

Fluorescent staining was performed on the lung of mice in each group, and the results are shown in Figure 22. In the figure, the green part represents type I collagen (Collagen I), the red part represents α-SMA, and the blue part represents DAPI. It can be seen that the mice in the PBS group and scrRNA group had more type I collagen and α-SMA, while mice in the experimental group had relatively less type I collagen and α-SMA, and in particular, mice in the Anti-miR-21 (5 mg/kg) group, Anti-miR-21 (10 mg/kg) group, Anti-miR-21+TGF-β1 siRNA (10 mg/kg) group had almost no expression of type I collagen and α-SMA.

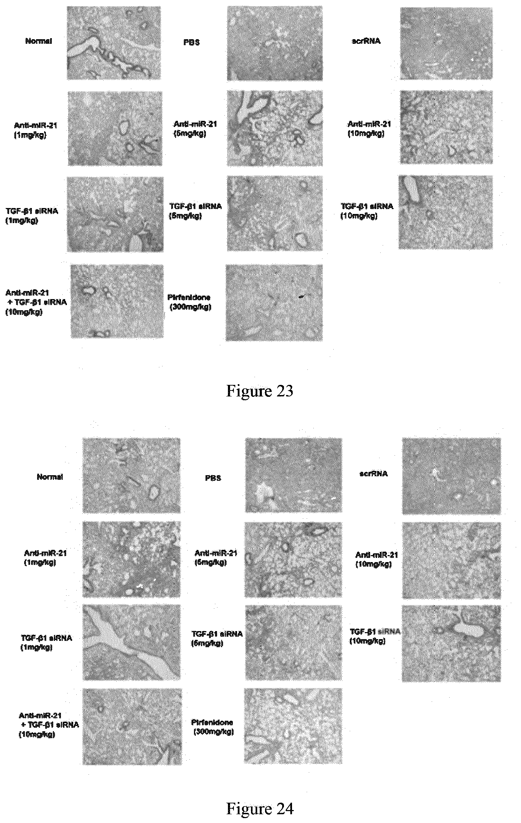

Masson's trichrome staining was performed on the lung of mice in each group, and the results are shown in Figure 23. It can be seen that the alveolar space of mice in the PBS group and scrRNA group was severely damaged, resulting in pulmonary interstitial collagen, while the experimental groups significantly alleviated these conditions.

H&E staining was performed on the lung of mice in each group, and the results are shown in Figure 24. It can be seen that mice in the PBS group and scrRNA group had widened alveolar space, infiltrated inflammatory cells and damaged alveolar structure, while the lung tissue of the experimental groups was relatively normal.

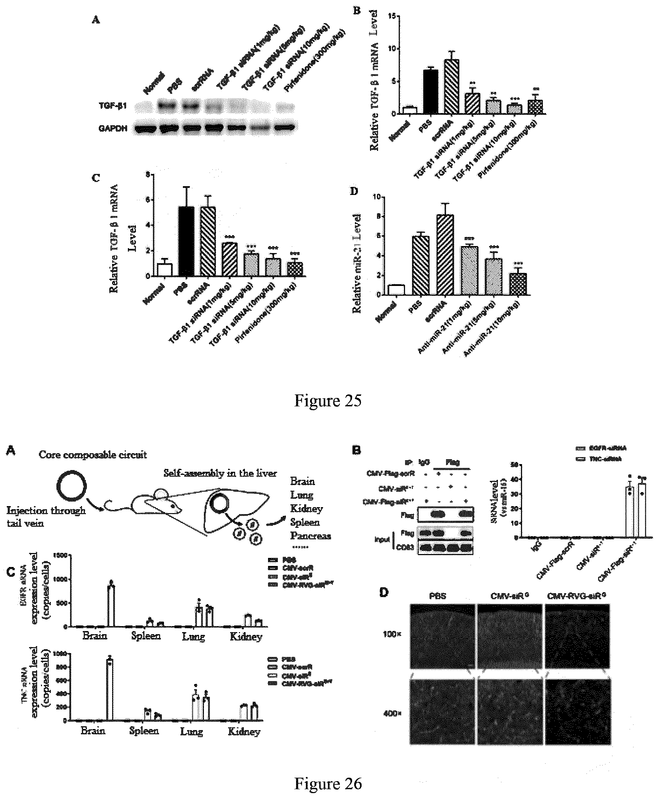

The TGF-β1 protein level and TGF-β1 mRNA level were detected in mice in the Normal group, PBS group, scrRNA group, TGF-β1 siRNA (1 mg/kg) group, TGF-β1 siRNA (5 mg/kg) group, TGF-β1 siRNA (10 mg/kg) group, and Pirfenidone (300 mg/kg) by western blot, and the results are shown in Figure 25A-Figure 25C. It can be seen that the TGF-β1 protein level and TGF-β1 mRNA level of mice in the TGF-β1 siRNA (10 mg/kg) group were the lowest, showing that after tail vein injection of the corresponding siRNA expression plasmid, TGF-β1 can be successfully delivered to the lung to function.

The relative miR-21 level was detected in mice in the Normal group, PBS group, scrRNA group, Anti-miR-21 (1 mg/kg) group, Anti-miR-21 (5 mg/kg) group, and Anti-miR-21 (10 mg/kg) group, and the results are shown in Figure 25D. It can be seen that the relative miR-21 level of mice in the Anti-miR-21 (10 mg/kg) group was the highest, showing that after tail vein injection of the corresponding antisense chain expression plasmid, the antisense chain of miR-21 can be successfully delivered to the lung to function.

The above experiments show that the use of the plasmid with high affinity for the liver to encapsulate the CMVsiR^{miR-21}, CMV-siR^{TGF-β1}, and CMV-siR^{miR-21+TGF-β1} circuits can significantly alleviate the degree of pulmonary fibrosis, with great drug potential and clinical research value.

### Example 7

On the basis of Example 1 or 2, use of an RNA delivery system in the manufacture of a medicament for treating a disease is provided in this example. The medicament is used for treating glioblastoma. This example will specifically illustrate the use of the RNA delivery system in the treatment of glioblastoma through the following experiments.

In the first experiment, we set up 5 experimental groups and 3 control groups. The experimental groups were CMV-siR^{E} group, CMV-siR^{T} group, CMV-RVG-siR^{E+T} group, CMV siR^{E+T} group, CMV-Flag-siR^{E+T} group, where "E" represents EGFR, "T" represents TNC, and the control groups were PBS group, CMV-scrR group, and CMV-Flag-scrR group. The specific experimental protocol is shown in Figure 26A.

The expression levels of CD63 protein and siRNA of mice in each group were detected. The results are shown in Figure 26B-Figure 26D, indicating that intravenous injection of the CMV RVG-siR^{E+T} circuit can deliver siRNA to the brain.

In the second experiment, we set up 2 experimental groups and 2 control groups. The experimental groups were CMV-RVG-siR^{E} group and CMV-RVG-siR^{E+T} group, and the control groups were PBS group and CMV-scrR group.

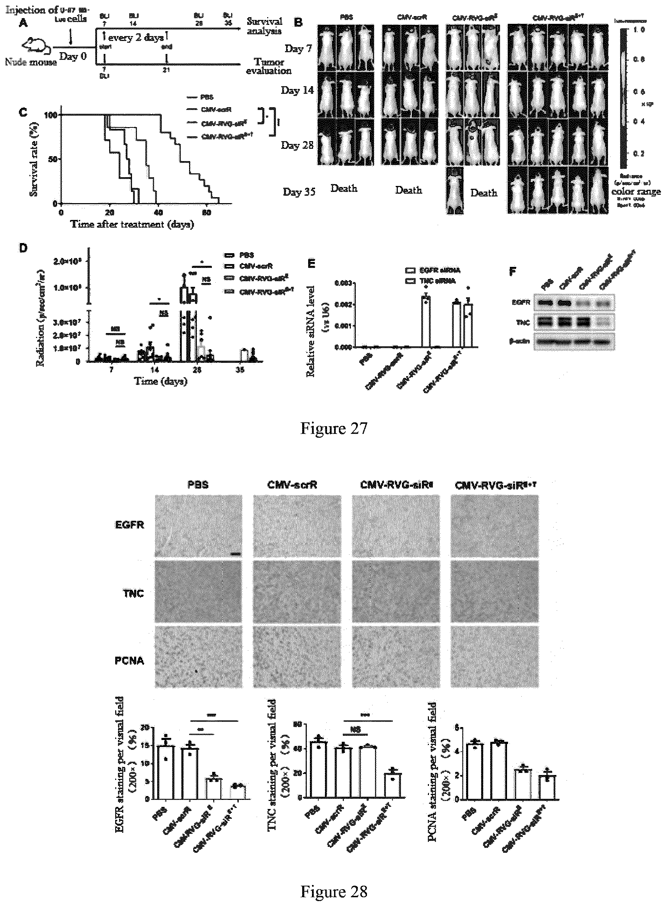

The specific experimental protocol is shown in Figure 27A. Mice were selected and injected with glioblastoma cells (U-87 MG-Luc cells). From the 7th day to the 21st day, mice were injected with PBS buffer/CMV-scrR/CMVRVG-siR^{E}/CMV-RVG-siR^{E+T} (5 mg/kg) every two days for treatment, and survival analysis and tumor evaluation were performed on the mice. On days 7, 14, 28 and 35, the mice were subjected to BLI in vivo imaging detection.

As shown in Figure 27B, the figure shows the comparison of mouse BLI in vivo imaging detection on days 7, 14, 28, and 35. It can be seen that mice in the CMV-RVG-siR^{E+T} group had the most significant inhibitory effect on glioblastoma.

As shown in Figure 27C, the figure shows the comparison of the survival rates of mice in each group. It can be seen that mice in the CMV-RVG-siR^{E+T} group had the longest survival time.

As shown in Figure 27D, the figure shows the comparison of fluorescence images of mice in each group, which was obtained using luciferase by in vivo bioluminescence imaging, where the ordinate reflects the intensity of lucifer fluorescence signal. Because the gene had been artificially integrated into the implanted tumors, the figure is able to reflect tumor progression. It can be seen that the tumors of mice in the control groups developed relatively rapidly, while the tumors of mice in the experimental group were suppressed to a great extent.

As shown in Figure 27E, the figure shows the comparison of the relative siRNA of mice in each group. It can be seen that the level of EGFR siRNA in mice in CMV-RVG-siR^{E} group was high, and the levels of both EGFR siRNA and TNC siRNA in mice in CMVRVG-siR^{E+T} were high.

As shown in Figure 27F, the figure shows the comparison of western blot results of mice in each group. It can be seen that mice in the PBS group, CMV-scrR group, and CMV-RVG-siR^{E} group had high levels of EGFR and TNC genes.

The above experimental data demonstrate that intravenous injection of CMV-RVG-siR^{E+T} plasmid can deliver siRNA to the brain and inhibit the growth of glioblastoma.

The brains of mice in each group were subjected to immunohistochemical staining, and the proportion of EGFR, TNC, and PCNA staining in each visual field was counted. The results are shown in Figures 28. It can be seen that mice in the CMV-RVG-siR^{E+T} group had the lowest EGFR, TNC, and PCNA contents in the brain, and mice in the CMV-RVG-siR^{E} group had low EGFR and PCNA contents in the brain. It can be seen that the injection of CMV-RVG-siR^{E} plasmid can inhibit the expression of EGFR and PCNA in the brain, and the injection of CMVRVG-siR^{E+T} plasmid can inhibit the expression of EGFR, TNC and PCNA in the brain.

### Example 8

On the basis of Example 1 or 2, use of an RNA delivery system in the manufacture of a medicament for treating a disease is provided in this example. The medicament is used for treating obesity. This example will specifically illustrate the use of the RNA delivery system in the treatment of obesity through the following two experiments.

In the first experiment, 2 experimental groups and 1 control group were set up. The experimental groups were CMV-siR^{P} group and CMV-RVG-siR^{P} group, and the control group was CMV-scrR group, where "P" means PTP1B.

Mice in the CMV-siR^{P} group, CMVRVG-siR^{P} group, and CMV-scrR group were injected with 5 mg/kg CMV-siR^{P} plasmid, CMV-RVG-siR^{P} plasmid, and CMV-scrR plasmid respectively, and then fluorescence microscope images of the hypothalamus and liver of mice in each group were obtained respectively. The results are shown in Figure 40 that PTP1B siRNA can be delivered to the hypothalamus.

In the second experiment, 2 experimental groups and 2 control groups were set up. The experimental groups were CMV-siR^{P} group and CMV-RVG-siR^{P} group, and the control groups were PBS group and CMV-scrR group.

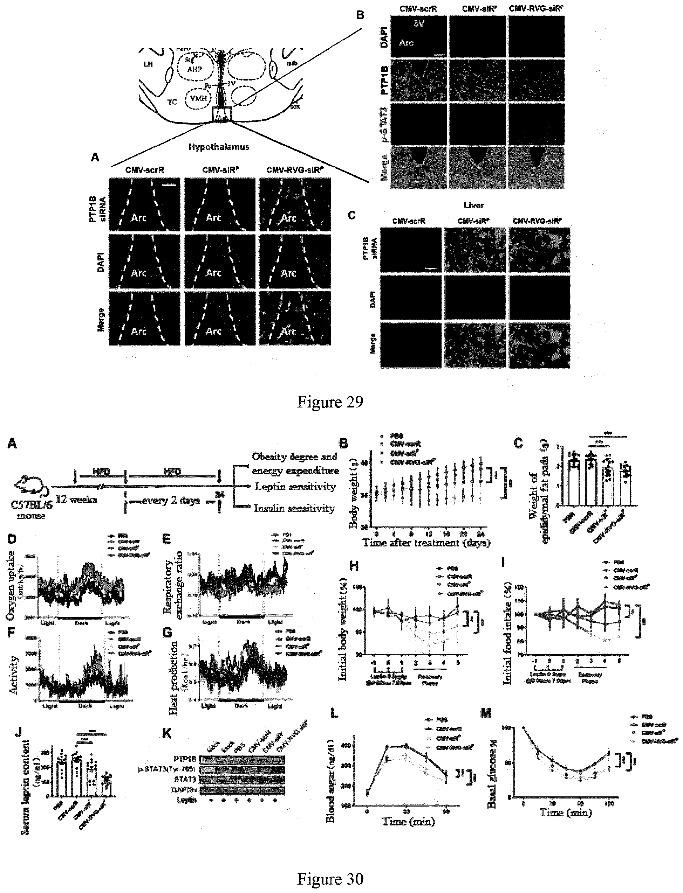

The specific experimental protocol is shown in Figure 30A. C57BL/6 mice were selected and injected with PBS buffer/CMV-scrR/CMV-siR^{P}/CMV-RVG-siR^{P} 12 weeks later, and once every two days for 24 days. Finally, the obesity degree, energy expenditure, leptin sensitivity, and insulin sensitivity of mice were counted.

As shown in Figure 30B, the figure shows the comparison of the body weights of mice in each group. It can be seen that the body weight of mice in the CMV-RVG-siR^{P} group was the most stable.

As shown in Figure 30C, the figure shows the comparison of the weights of the epididymal fat pads of mice in each group. It can be seen that the weight of the epididymal fat pads of mice in the CMV RVG-siR^{P} group was the lightest.

The oxygen consumption, respiratory exchange ratio, activity volume, and heat production of differently treated mice were continuously detected for 72 h using metabolic cages, and then the average values were drawn for statistical analysis. The results are shown in Figure 30D-Figure 30G. The results show that the CMV-RVG-siR^{P} plasmid can effectively increase the oxygen consumption of mice, which means that mice in this group were in a high-energy metabolic state compared with mice in other groups. Normal mice mainly use glucose as their own energy source. CMV-RVG-siR^{P} plasmid can reduce the respiratory exchange ratio of mice, which means that mice in this group were more inclined to use protein as their own energy source compared with mice in other groups. The activity level of mice injected with CMV-RVG-siR^{P} plasmid was significantly increased. Moreover, the heat production of mice in the CMV-RVG-siR^{P} group was also significantly increased.

As shown in Figure 30H, this figure shows the comparison of the initial body weight curves of mice in each group. It can be seen that the mice in the CMV-RVG-siR^{P} group had the lightest body weight.

As shown in Figure 30I, this figure shows the comparison of the initial food intake curves of mice in each group. It can be seen that the mice in the CMV-RVG-siR^{P} group had the least food intake.

As shown in Figure 30J, this figure shows the comparison of serum leptin levels in mice of each group. It can be seen that the serum leptin levels of the mice in the CMV-RVG-siR^{P} group were the lowest.

As shown in Figure 30K, this figure shows the comparison of western blot results of mice in each group. It can be seen that the PTP1B protein content of mice in the CMV-RVG-siR^{P} group was the lowest.

As shown in Figure 30L, this figure shows the comparison of blood glucose change curves of mice in each group. It can be seen that the blood glucose content of mice in the CMV-RVG-siR^{P} group was the lowest.

As shown in Figure 30M, this figure shows the comparison of the basal glucose change curves of mice in each group. It can be seen that the basal glucose content of mice in the CMV RVG-siR^{P} group was the lowest.

From the above experiments, it can be concluded that intravenous injection of CMV RVG-siR^{P} plasmid can reduce obesity in obese mouse models.

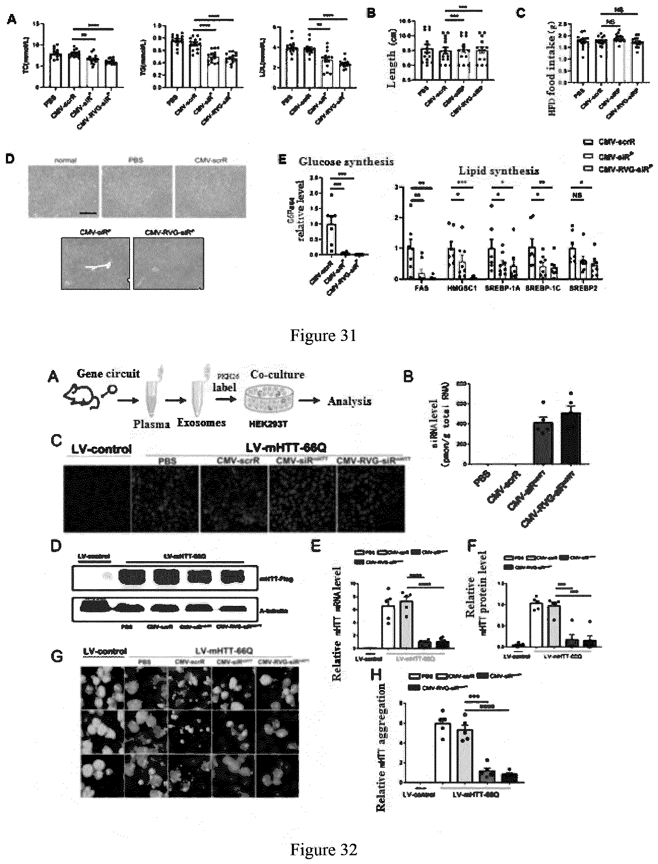

The serum total cholesterol (TC), triglycerides (TG), and low-density lipoprotein (LDL) of mice in each group were measured, and the results are shown in Figure 31A. It can be seen that the mice in the CMV-RVG-siR^{P} group had the lowest TC, TG, and LDL contents.

The body length of mice in each group was measured, and the results are shown in Figure 31B. It can be seen that the body length of mice in the four groups was almost the same.

The HFD food intake of mice in each group was counted, and the results are shown in Figure 31C. It can be seen that the HFD food intake of mice in the four groups was almost the same.

The liver tissue of mice in each group was collected after treatment and compared with the normal control. The results are shown in Figure 31D. Obvious pathological characteristics of fatty liver can be seen in the liver tissue pathological sections of mice in the PBS group and CMV scrR group, while mice in the CMV-siR^{P} group had milder fatty liver.

The above experiments show that intravenous injection of CMVRVG-siR^{P} plasmid can reduce fatty liver in obese mice.

### Example 9

On the basis of Example 1 or 2, use of an RNA delivery system in the manufacture of a medicament for treating a disease is provided in this example. The medicament is used for treating Huntington's disease. This example will specifically illustrate the use of the RNA delivery system in the treatment of Huntington's disease through the following five experiments.

In the first experiment, 2 experimental groups and 2 control groups were set up. The experimental groups were CMV-siR^{mHTT} group and CMV-RVG-siR^{mHTT} group, and the control groups were PBS group and CMV-scrR group.

The experimental protocol is shown in Figure 32A. Mice with Huntington's disease in the CMV-siR^{mHTT} group, CMV-RVG-siR^{mHTT} group, PBS group, and CMV-scrR group were injected with CMV-siR^{mHTT} plasmid, CMV-RVG-siR^{mHTT} plasmid, PBS solution, and CMV-scrR plasmid through the tail vein, respectively. The plasma exosomes were then isolated, labeled with PKH26 dye, and co-cultured with cells to observe the absorption of exosomes by cells.

As shown in Figure 32B, the figure shows the comparison of siRNA levels in plasma exosomes of mice in each group. It can be seen that the levels of siRNA in plasma exosomes of mice in the two experimental groups were higher.

After injecting plasmid/solution into mice in each group, plasma exosomes were extracted, labeled with PKH26, co-cultured with cells and photographed using a confocal microscope. The results are shown in Figure 32C, showing that siRNA-encapsulated exosomes entered cells.

After co-culturing the extracted plasma exosomes from mice in each group with cells, the changes in HTT protein levels and mRNA levels of mice in each group were detected. As shown in Figure 32D-Figure 32F, the results show that CMVsiR^{mHTT} and CMV-RVG-siR^{mHTT} can reduce HTT protein levels, indicating that siRNA assembled into exosomes can still exert gene silencing function.

After co-culturing the extracted plasma exosomes from mice in each group with cells, the aggregation of HTT protein of mice in each group was observed and counted. As shown in Figure 32G-Figure 32H, the results show that CMV-siR^{mHTT} and CMV-RVG-siR^{mHTT} can reduce the aggregation of pathological HTT proteins in the Huntington HTT aggregation cell model, indicating that siRNA assembled into exosomes can still exert gene silencing function and can effectively reduce the aggregation of mutant proteins.

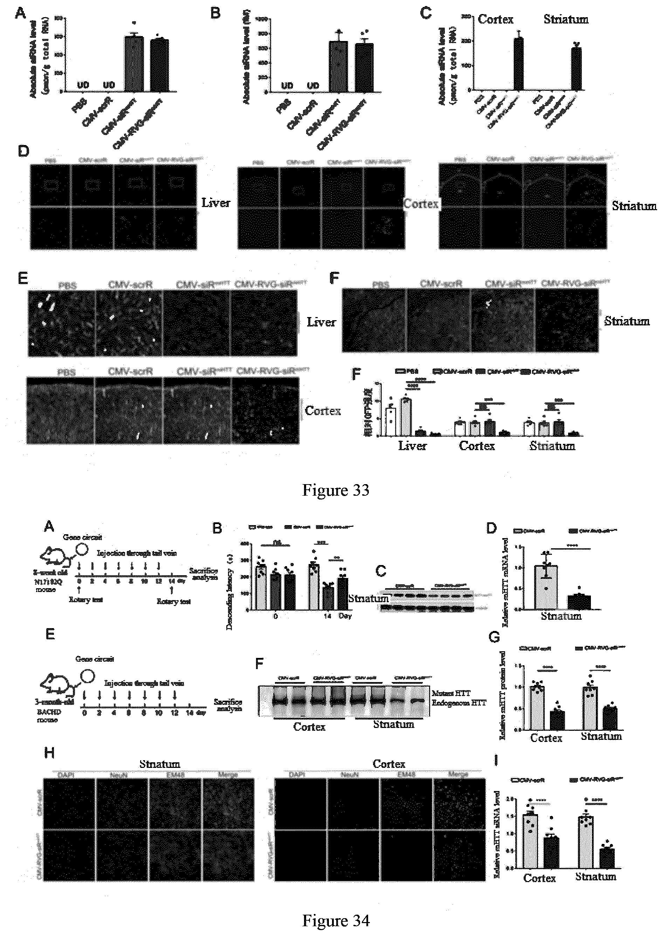

The absolute siRNA expression levels in the liver, plasma, cortex, and striatum of mice in each group were detected respectively. As shown in Figure 33A, this figure shows the comparison of the absolute siRNA levels in the liver of mice in each group. It can be seen that the absolute siRNA levels of mice in the CMV-siR^{mHTT} group and CMV-RVG-siR^{mHTT} group were higher. As shown in Figure 33B, this figure shows the comparison of the absolute siRNA levels in the plasma of mice in each group. It can be seen that the absolute siRNA levels of mice in the CMV-siR^{mHTT} group and CMV-RVG-siR^{mHTT} group were higher. As shown in Figure 33C, this figure shows the comparison of the absolute siRNA levels in the cortex and striatum of mice in each group. It can be seen that the absolute siRNA levels of mice injected with CMV-RVG-siR^{mHTT} were higher.

As shown in Figure 33D, this figure shows the in situ hybridization of mouse liver, cortex and striatum tissue in each group. It can be seen that the liver tissue sections of mice in the CMV siR^{mHTT} group and CMV-RVG-siR^{mHTT} group showed obvious fluorescence, and the cortex and striatum tissue sections of mice in the CMV-RVG-siR^{mHTT} group showed obvious fluorescence. This shows that RVG can guide exosomal siRNA to enter and pass through the blood-brain barrier and exert its function.

In the second experiment, 2 experimental groups and 2 control groups were set up. The experimental groups were CMV-siR^{GFP} group and CMV-RVG-siR^{GFP} group, and the control groups were PBS group and CMV-scrR group. GFP transgenic mice in the CMV-siR^{GFP} group, CMV RVG-siR^{GFP} group, PBS group, and CMV-scrR group were injected with CMV-siR^{GFP} plasmid, CMV-RVG-siR^{GFP} plasmid, PBS solution, and CMV-scrR plasmid through the tail vein, respectively.

As shown in Figure 33E and Figure 33F, the figure shows the sections of the liver, cortex and striatum of mice in each group. It can be seen that the transgenic mice injected with CMV siR^{GFP}/CMV-RVG-siR^{GFP} in the liver had reduced GFP fluorescence level, and the transgenic mice injected with CMV-RVG-siR^{GFP} in the cortex and striatum had reduced GFP fluorescence level. This shows that RVG can guide exosomal siRNA to enter and pass through the blood-brain barrier and exert its function.

In the third experiment, two experimental groups and one control group were set up. The experimental groups were CMV-siR^{mHTT} group and CMV-RVG-siR^{mHTT} group, and the control group was CMV-scrR group.

The experimental protocol is shown in Figure 34A. The 8-week-old N17182Q mice were selected, and mice with Huntington's disease in the CMV-siR^{mHTT} group, CMV-RVG-siR^{mHTT} group, and the CMV-scrR group were injected with CMV-siR^{mHTT} plasmid, CMV-RVG-siR^{mHTT} plasmid and CMV-scrR plasmid through the tail vein, respectively. Rotation test was performed on days 0 and 14, and mice were sacrificed after 14 days for analysis.

As shown in Figure 34B, this figure shows the comparison of the descending latency of wild-type mice and mice in the CMV-scrR group and CMV-RVG-siR^{mHTT} group. It can be seen that on day 0, the descending latency of mice in the CMV-scrR group and CMV-RVG-siR^{mHTT} group was relatively consistent. On day 14, the descending latency of mice in the CMV-scrR group was the shortest.

As shown in Figure 34C and Figure 34D, Figure 34C shows the western blot results of the striatum of mice in the CMV-scrR group and CMV-RVG-siR^{mHTT} group, and Figure 34D shows the comparison of the relative mHTT mRNA levels in the striatum of mice in the CMV-scrR group and CMV-RVG-siR^{mHTT} group. It can be seen that mice in the CMV-scrR group had higher N171-mHTT protein level and higher relative mHTT mRNA level in the striatum.

In the fourth experiment, one experimental group and one control group were set up. The experimental group was CMV-RVG-siR^{mHTT} group, and the control group was CMV-scrR group.

The experimental protocol is shown in Figure 34E. The 3-month-old BACHD mice were selected. Mice with Huntington's disease in the CMV-RVG-siR^{mHTT} group and CMV-scrR group were injected with CMV-RVG-siR^{mHTT} plasmid and CMV-scrR plasmid through the tail vein, respectively, and were sacrificed after 14 days for analysis.

As shown in Figure 34F, Figure 34F shows the western blot results of the cortex and striatum of mice in the CMV-scrR group and CMV-RVG-siR^{mHTT} group, and it can be seen that mice in the CMV-RVG-siR^{mHTT} group had less mutant HTT and endogenous HTT in the cortex and striatum.

As shown in Figure 34G, Figure 34G shows the comparison of the relative mHTT protein levels in the cortex and striatum of mice in the CMV-scrR group and the CMV-RVG-siR^{mHTT} group. It can be seen that whether in the mouse cortex or the striatum, the relative mHTT protein levels of mice in the CMV-RVG-siR^{mHTT} group were lower.

As shown in Figure 34H and Figure 34I, Figure 34H shows the immunofluorescence images of mice in the CMV-scrR group and CMV-RVG-siR^{mHTT} group, and Figure 34I shows the comparison of the relative mHTT mRNA levels in the cortex and striatum of mice in the CMV scrR group and the CMV-RVG-siR^{mHTT} group. It can be seen that whether in the mouse cortex or the striatum, the relative mHTT mRNA levels of mice in the CMV-RVG-siR^{mHTT} group were lower.

The above experiments demonstrate that intravenous injection of MV-RVG-siR^{mHTT} plasmid helps suppress mHTT in the striatum and cortex, thereby improving exercise capacity and alleviating neuropathology in HD mice.

In the fifth experiment, one experimental group and one control group were set up. The experimental group was the CMV-RVG-siR^{mHTT} group, and the control group was the CMV-scrR group.

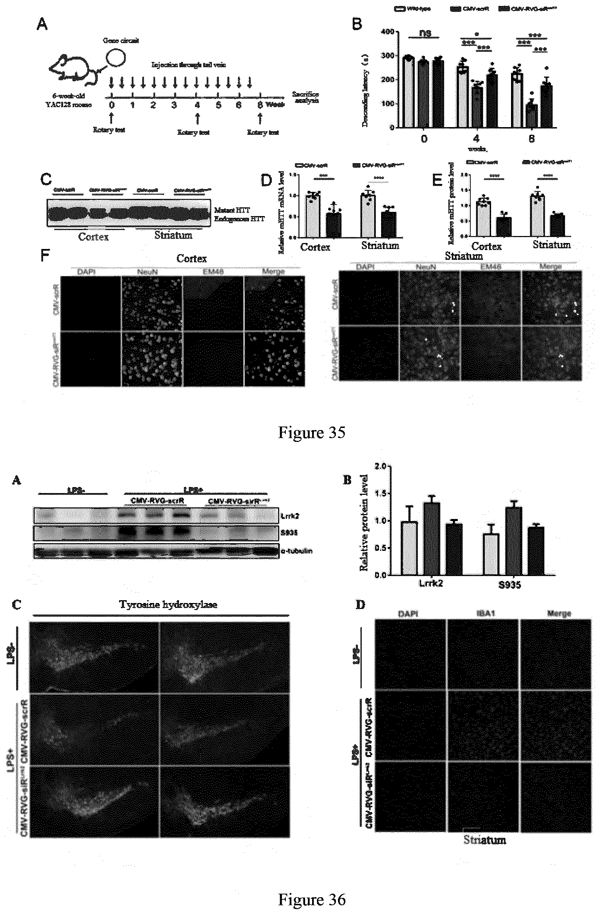

The experimental protocol is shown in Figure 35A. The 6-week-old YAC128 mice were selected. Mice with Huntington's disease in the CMV-RVG-siR^{mHTT} group and CMV-scrR group were injected with CMV-RVG-siR^{mHTT} plasmid and CMV-scrR plasmid through the tail vein, respectively. The rotation test was performed on the 0th day, 4th week, and 8th week of the experiment, and then the mice were sacrificed for analysis.

As shown in Figure 35B, this figure shows the comparison of the descending latency of wild-type mice and mice in the CMV-RVG-siR^{mHTT} group and CMV-scrR group. It can be seen that on day 0, the descending latency of mice in the CMV-RVG-siR^{mHTT} group and CMV-scrR group was relatively consistent. At the fourth and eighth weeks, the descending latency of mice in the CMV-scrR group was the shortest.

As shown in Figure 35C, this figure shows the western blot results of the cortex and striatum of mice in the CMV-RVG-siR^{mHTT} group and CMV-scrR group. It can be seen that mice in the CMV-RVG-siR^{mHTT} group had lower mutant HTT and endogenous HTT contents in the cortex, and had lower mutant HTT and higher endogenous HTT contents in the striatum.

As shown in Figure 35D and Figure 35E, the two figures show the relative mHTT mRNA levels and the relative mHTT protein levels in the cortex and striatum of mice in the CMV-RVG-siR^{mHTT} group and CMV-scrR group. It can be seen that the relative mHTT mRNA levels and the relative mHTT protein levels of mice in the CMV-RVG-siR^{mHTT} group were relatively lower in both cortex and striatum.

As shown in Figure 35F, this figure show immunofluorescence images of the cortex and striatum of mice in the CMV-RVG-siR^{mHTT} group and CMV-scrR group. It can be seen that the expression levels of NeuN and EM48 of mice in the CMV-RVG-siR^{mHTT} group were lower than those in the CMV-scrR group.

The above experiments can demonstrate that intravenous injection of CMV-RVG-siR^{mHTT} plasmid can help reduce mHTT protein and toxic aggregates in the striatum and cortex, thereby improving behavioral defects and neuropathology in the striatum and cortex.

### Example 10

On the basis of Example 1 or 2, use of an RNA delivery system in the manufacture of a medicament for treating a disease is provided in this example. The medicament is used for treating Parkinson's disease. This example will specifically illustrate the use of the RNA delivery system in the treatment of Parkinson's disease through the following experiments.

In this experiment, LRRK2R1441G transgenic mice were selected for the experiment when they were 3 months old, and an LPS intervention group and an LPS non-intervention group were set up. The LPS intervention group was treated with CMV-scrR/CMV-RVG-siR^{LRRK2} 7 days after LPS intervention.

As shown in Figure 36A and Figure 36B, Figure 36A shows the western blot results of LRRK2R1441G transgenic mice injected with CMV-scrR/CMV-RVG-siR^{LRRK2}, and Figure 36B shows the protein grayscale analysis of LRRK2R1441G transgenic mice injected with CMV-scrR/CMV-RVG-siR^{LRRK2}. It can be seen that the levels of LRRK2 protein and S935 protein in mice injected with CMV-RVG-siR^{LRRK2} were reduced, indicating that CMV-RVG-siR^{LRRK2} can cross the blood-brain barrier and reduce the expression of deep brain proteins after siRNA was released from the liver and assembled into exosomes.

As shown in Figure 36C, this figure shows immunofluorescence images of TH+ neurons in the substantia nigra area of LRRK2R1441G transgenic mice injected with CMV-scrR/CMV-RVG-siR^{LRRK2}. The results show that the loss of TH neurons was rescued in mice injected with CMV-RVG-siR^{LRRK2}, indicating that CMV-RVG-siR^{LRRK2} can cross the blood-brain barrier and enter the deep brain to function after siRNA was released from the liver and assembled into exosomes.

As shown in Figure 36D, this figure shows immunofluorescence images of microglial activation levels in LRRK2R1441G transgenic mice injected with CMV-scrR/CMV-RVG-siR^{LRRK2}. The results show that microglia activation can be inhibited in mice injected with CMV-RVG-siR^{LRRK2}, indicating that CMV-RVG-siR^{LRRK2} can cross the blood-brain barrier and enter the deep brain to function after siRNA was released from the liver and assembled into exosomes.

The above experiments can demonstrate that intravenous injection of CMV-RVG-siR^{LRRK2} plasmid helps suppress LRRK2 in dopaminergic neurons, thereby reducing the development of neuropathology in mice with Parkinson's disease.

### Example 11

To further investigate this critical issue and elucidate the pharmacokinetics and pharmacodynamics of self-assembled siRNA in vivo, we conducted a more detailed study in Macaca fascicularis, a well-known non-human primate model used in safety assessment studies. With ethical approval, we used 4 adult macaques for intravenous injection of 5 mg/kg CMV-siR^{E} plasmid, and blood samples were collected before injection or at different time points after injection. One month later, these macaques were intravenously injected with 5 mg/kg CMV-siR^{E} plasmid daily for a total of 5 times, and blood samples were collected before injection or at different time points after the last injection.

As shown in Figure 37, Figure 37A shows siRNA concentration changes in whole blood of Macaca fascicularis with a single injection. Figure 37B shows siRNA concentration changes in whole blood of Macaca fascicularis with multiple injections. It can be seen that the concentration of siRNA in Macaca fascicularis with a single injection reached a peak 6 h after intravenous injection, and then decreased. The concentration of siRNA in Macaca fascicularis with multiple injections reached a peak 3 h after intravenous injection, and then decreased. The siRNA concentration of Macaca fascicularis with multiple injections decreased more slowly.

The above experiments can show that CMV-siR^{E} plasmid can be safe and effective for primates such as Macaca fascicularis, with broad application prospects.

The terms "upper", "lower", "front", "back", "left", "right", etc. used herein are only used to express the relative positional relationship between related parts, but not to limit the absolute position of these related parts.

The terms "first", "second", etc. used herein are only used to distinguish each other, but do not indicate the degree of importance, order, or the prerequisite for each other's existence.

The terms "equal", "identical", etc. used herein are not limitations in a strict mathematical and/or geometric sense, but also include errors that are understandable by those skilled in the art and allowed in manufacturing or use.

Unless otherwise stated, numerical ranges herein include not only the entire range within both endpoints, but also several subranges subsumed therein.

The preferred specific embodiments and examples of the present application have been described in detail above in conjunction with the accompanying drawings. However, the present application is not limited to the above-mentioned embodiments and examples. Within the scope of knowledge possessed by those skilled in the art, various changes can be made without departing from the concept of the present application.

## Claims

1. An RNA plasmid delivery system, wherein the system comprises a plasmid carrying a RNA fragment required to be delivered, the plasmid can be enriched in an organ tissue of a host, and spontaneously forms a complex structure containing the RNA fragment in the organ tissue of the host, the complex structure can enter and be combined with a target tissue to deliver the RNA fragment into the target tissue.

2. The RNA plasmid delivery system as claimed in claim 1, wherein the RNA fragment comprises one, two or more specific RNA sequences of medical significance, which are siRNA, shRNA, or miRNA sequences of medical significance.

3. The RNA plasmid delivery system as claimed in claim 1, wherein the plasmid further comprises a promoter and a targeting tag, the targeting tag can form a targeting structure of the complex structure in the organ tissue of the host, the targeting structure is located on the surface of the complex structure, and the complex structure can search for and be combined with the target tissue through the targeting structure to deliver the RNA fragment into the target tissue.

4. The RNA plasmid delivery system as claimed in claim 3, wherein the plasmid comprises any one or a combination of the following circuits: promoter-RNA fragment, promoter-targeting tag, promoter-RNA fragment-targeting tag; wherein each of the plasmid comprises at least one RNA fragment and one targeting tag, the RNA fragment and targeting tag are located in the same circuit or in different circuits.

5. The RNA plasmid delivery system as claimed in claim 4, wherein the plasmid further comprises a flanking sequence, a compensation sequence, and a loop sequence that facilitate the correct folding and expression of the circuit, wherein the flanking sequence comprises a 5' flanking sequence and a 3' flanking sequence; and
the plasmid comprises any one or a combination of the following circuits: 5' promoter-5' flanking sequence-RNA fragment-loop sequence-compensation sequence-3' flanking sequence, 5'-promoter-targeting tag, and 5' promoter-targeting tag-5' flanking sequence-RNA fragment-loop sequence-compensation sequence-3' flanking sequence.

6. The RNA plasmid delivery system as claimed in claim 5, wherein the 5' flanking sequence has a sequence that is identical to or more than 80% homologous with ggatcctggaggcttgctgaaggctgtatgctgaattc;
the loop sequence has a sequence that is identical to or more than 80% homologous with gttttggccactgactgac;
the 3' flanking sequence has a sequence that is identical to or more than 80% homologous with accggtcaggacacaaggcctgttactagcactcacatggaacaaatggcccagatctggccgcactcgag; and
the compensation sequence has a reverse complementary sequence to the RNA fragment in which any 1 to 5 bases have been deleted.

7. The RNA plasmid delivery system as claimed in claim 4, wherein in the presence of at least two circuits in the plasmid, adjacent circuits are connected through a sequence consisted of sequences 1-3;
wherein, sequence 1 is CAGATC, sequence 2 is a sequence of 5-80 bases, and sequence 3 is TGGATC.

8. The RNA plasmid delivery system as claimed in claim 7, wherein in the presence of at least two circuits in the plasmid, adjacent circuits are connected through sequence 4 or a sequence with homology greater than 80% with sequence 4;
wherein, sequence 4 is
CAGATCTGGCCGCACTCGAGGTAGTGAGTCGACCAGTGGATC.

9. The RNA plasmid delivery system as claimed in claim 1, wherein the organ tissue is liver, and the complex structure is an exosome.

10. The RNA plasmid delivery system as claimed in claim 3, wherein the targeting tag is selected from the group consisting of a targeting peptide or protein with a targeting function.

11. The RNA plasmid delivery system as claimed in claim 10, wherein the targeted peptide is selected from the group consisting of RVG targeting peptide, GE11 targeting peptide, PTP targeting peptide, TCP-1 targeting peptide, and MSP targeting peptide; and
the targeting protein is selected from the group consisting of RVG-LAMP2B fusion protein, GE11-LAMP2B fusion protein, PTP-LAMP2B fusion protein, TCP-1-LAMP2B fusion protein, and MSP-LAMP2B fusion protein.

12. The RNA plasmid delivery system as claimed in claim 2, wherein the RNA sequence is 15-25 nucleotides in length.

13. The RNA plasmid delivery system according to claim 12, wherein the RNA sequence has a sequence that is identical to, or more than 80% homologous with, or of a nucleic acid molecule encoding a sequence selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, siRNA of α-synuclein gene, and a combination thereof.

14. The RNA plasmid delivery system as claimed in claim 1, wherein the delivery system is a delivery system for application in a mammal including human.

15. Application of the RNA plasmid delivery system according to any one of claims 1 to 14 in the manufacture of a medicament for treating a disease.

16. The application according to claim 15, wherein the medicament is administered by oral administration, inhalation, subcutaneous injection, intramuscular injection, or intravenous injection.

17. The application according to claim 15, wherein the disease is a cancer, pulmonary fibrosis, colitis, obesity, cardiovascular disease caused by obesity, type 2 diabetes, Huntington's disease, Parkinson's disease, myasthenia gravis, Alzheimer's disease, or graft-versus-host disease.
